# EUROPÄISCHE PATENTSCHRIFT

(11) **EP 3 382 377 B1**
(45) Veröffentlichungstag und Bekanntmachung des Hinweises auf die Patenterteilung: **29.03.2023**
(21) Anmeldenummer: 18159395.5
(22) Anmeldetag: 01.03.2018
(51) Int. Cl.: G01N 21/25, G01N 21/27, G01N 21/51, G01N 21/17, G01N 21/77, G01N 21/80

(54) **VORRICHTUNG, VERFAHREN UND SYSTEM ZUR ERFASSUNG MINDESTENS EINER VERÄNDERLICHEN WÄHREND EINES BIOLOGISCHEN, CHEMISCHEN ODER BIOCHEMISCHEN PROZESSES**
DEVICE, METHOD AND SYSTEM FOR DETECTING AT LEAST ONE VARIABLE DURING A BIOLOGICAL, CHEMICAL OR BIOCHEMICAL PROCESS
DISPOSITIF, PROCÉDÉ ET SYSTÈME DE DÉTERMINATION D'AU MOINS UNE VARIABLE LORS D'UN PROCESSUS BIOLOGIQUE, CHIMIQUE OU BIOCHIMIQUE

(30) Priorität: 31.03.2017 DE 102017107033
(43) Veröffentlichungstag der Anmeldung: 03.10.2018
(73) Patentinhaber: PreSens - Precision Sensing GmbH, 93053 Regensburg (DE)
(72) Erfinder: UDE, Christian Matthias, 30880 Laatzen (DE)
(74) Vertreter: Reichert & Lindner Partnerschaft Patentanwälte

(56) Entgegenhaltungen:
- EP-B1- 1 730 494
- US-A1- 2005 176 155
- US-B2- 7 339 671

## Beschreibung

Die Erfindung betrifft eine Vorrichtung zur Erfassung mindestens einer Veränderlichen von mehreren flüssigen Proben im mehreren Behältnissen während eines biologischen, chemischen oder biochemischen Prozesses.

Ferner betrifft die Erfindung ein Verfahren zur Erfassung mindestens einer Veränderlichen von mehreren flüssigen Proben in mehreren Behältnissen während eines biologischen, chemischen oder biochemischen Prozesses.

Auch betrifft die Erfindung ein System zur Erfassung mindestens einer Veränderlichen von mehreren flüssigen Proben in mehreren Behältnissen während eines biologischen, chemischen oder biochemischen Prozesses.

Die europäische Patentschrift EP 1 730 494 B1 offenbart ein Verfahren und eine Vorrichtung zur Erfassung von Prozessparametern von Reaktionsflüssigkeiten in mehreren Mikroreaktoren, die zumindest bis zur Beendigung der Reaktion in sämtlichen Mikroreaktoren kontinuierlich geschüttelt werden. Die Prozessparameter in den Mikroreaktoren werden während der Reaktion mithilfe mindestens einer Sensoroptik erfasst. Die Sensoroptik wird während der Erfassung der Werte eines Prozessparameters, zum Beispiel bei der Erfassung eines momentanen Wertes der Eigenfluoreszenz der Reaktionsflüssigkeit nicht bewegt. Die dabei auftretende Relativbewegung zwischen den geschüttelten Mikroreaktoren und jeder Sensoroptik ist unproblematisch, wenn die elektromagnetische Strahlung jeder Sensoroptik während der Erfassung der Prozessparameter in einem der Mikroreaktoren ausschließlich in diesen Mikroreaktor eingeleitet wird und die von der Reaktionsflüssigkeit ausgehende Strahlung ausschließlich auf den Sensor der zugehörigen Sensoroptik trifft. Die Messung erfolgt in kontinuierlich geschüttelten Reaktoren, wobei jede unter den Mikroreaktoren der Mikrotiterplatte ausgerichtete Sensoroptik zumindest während der Erfassung der Prozessparameter nicht bewegt wird, so dass die geschüttelten Mikroreaktoren sich relativ zu jeder Sensoroptik bewegen können.

Das US-Patent 7,339,671 B2 offenbart eine Vorrichtung und ein Verfahren zur Echtzeit- und Online-Überwachung des Zellwachstums und der Zellkonzentration in einer dynamischen Zellkulturumgebung mit Techniken, die Rauschen von Umgebungslicht, ungleichmäßige Streuverteilung sowie Blasen- und Grenzflächenreflexionseffekte in der dynamischen Umgebung unterdrücken. Die Vorrichtung umfasst mindestens einen Erlenmeyerkolben, der auf einer Schütteleinrichtung mit einem entsprechenden Messystem angeordnet ist.

Das US-Patent 8,405,033 B2 offenbart einen Sensor für die schnelle Detektion einer Teilchenkonzentration in einer Flüssigkeit. Die Teilchenkonzentration wird dabei durch die Wand eines Gefäßes gemessen. Verschiedenartige Gefäßtypen können verwendet werden. Der Sensor umfasst eine oder mehrere Lichtquellen und einen oder mehrere Sensoren, die in einem Sensorgehäuse untergebracht sind. Gemäß einer möglichen Ausführungsform kann das Gefäß auch eine Well-Platte sein. Außerhalb eines jeden Wells ist dem Boden eines jeden Wells eine Lichtquelle und ein Sensor zugeordnet.

Das US-Patent 8,603,772 B2 offenbart ein Verfahren und eine Vorrichtung zur Detektion der Teilchengröße und/oder der Teilchenkonzentration. Hierzu sind ein oder mehrere Lichtquellen und ein oder mehrere Detektoren in einem Gehäuse untergebracht, das mit einem zu untersuchenden Medium in Kontakt ist. Die Vorrichtung ist auch für die nicht-invasive Messung von Biomasse in einem Bioreaktor geeignet.

Das US-Patent 6,673,532 B2 offenbart einen Bioreaktor, der eine nicht-invasive optischchemische Detektionsmethode benutzt. Eine Lichtquelle regt einen optisch-chemischen Sensor an, dessen optische Reaktion von einem Detektor gemessen wird. Gemäß einer Ausführungsform ist jedem Reaktor eine LED und ein Detektor zugeordnet. Die Beleuchtung erfolgt hier durch eine Seitenwand des Behältnisses. Die Detektion erfolgt durch eine andere Seitenwand des Behältnisses.

Das US-Patent 7,339,671 B2 offenbart ein Verfahren und eine Vorrichtung zur Real-Time und online-Überwachung des Zellwachstums und der Konzentration in einer dynamischen Zellkulturumgebung. Es werden Techniken angewendet, die das Rauschen des Umgebungslichts, uneinheitliche Verteilung der Streuung und Effekte der Reflexion in einer dynamischen Umgebung unterdrücken.

Das deutsche Patent DE 10 2014 001 284 B3 offenbart eine Vorrichtung und ein Verfahren zur Bestimmung der optischen Dichte und/oder der Veränderung der optischen Dichte eines Reaktionsgemisches in einem geschüttelten Reaktor. Dabei gelangt Licht von mindestens einer Lichtquelle in das Reaktionsgemisch. Das Reaktionsgemisch verlassende Licht wird durch mindestens einen Lichtsensor erfasst. Während der Erfassung des Lichtes durch den mindestens einen Lichtsensor werden der Reaktor und das Reaktionsgemisch geschüttelt. Die Erfassung des Lichtes durch den mindestens einen Lichtsensor erfolgt mit einer Frequenz, sodass die Schüttelfrequenz kein ganzzahliges Vielfaches der Erfassungsfrequenz ist und mindestens zwei in einem bestimmten zeitlichen Intervall von mindestens einem Lichtsensor erfassten Messpunkte zu einer Messreihe zusammengefasst werden. Das Reaktionsgemisch wird kontinuierlich geschüttelt. Es besteht zwischen Reaktor, Lichtquellen und Lichtsensoren keine Relativbewegung während der Aufnahme einer Messreihe.

Die internationale Patentanmeldung WO 2016/066156 betrifft eine mobile photometrische Messvorrichtung mit mindestens einem Messmodul, das aus einer Lichtquelle, einem Detektor und einem Optikgerüst mit einer Optik mit integrierten Filtereigenschaften besteht. Ebenso können eine Optik und mindestens ein Filter vorgesehen sein. Diese Komponenten sind auf einer Platine, in einem Gehäuse angeordnet und/oder mit einem Baustein verschaltet. Ferner betrifft die Erfindung ein mobiles photometrisches Messverfahren an Mikrotiterplatten mit Gittersensoren.

Die deutsche Offenlegungsschrift DE 10 2011 000 891 A1 offenbart ein Verfahren und eine Vorrichtung zur Bestimmung mindestens einer Veränderlichen einer sich in einem bewegten Behältnis befindlichen Probe. Das Behältnis wird in definierter Weise bewegt, und die mindestens eine Veränderliche bestimmt. Dabei wird die Bestimmung der mindestens einen Veränderlichen zeitlich mit einem durch die Bewegung des Behältnisses sich ergebenden Bewegungszustand der Probe abgestimmt. Zur Durchführung des Verfahrens ist ein Trageelement für das Behältnis vorgesehen, wobei mit dem Trageelement eine definierte Bewegung ausführbar ist. Ebenso ist ein Messsystem vorgesehen, durch welches mindestens eine Veränderliche der Probe bestimmbar ist. Es ist mindestens ein Synchronisiermittel vorgesehen, durch welches die Bestimmung der mindestens einen Veränderlichen mit einem durch die Bewegung des Behältnisses erzeugbaren Bewegungszustand der Probe zeitlich abstimmbar ist.

Die US-Patentanmeldung US 2005/0176155 A1 offenbart einen Reaktor aus einer Vielzahl von Mikrotiterplatten. Mit den einzelnen Wells der Mikrotiterplatte kann der Sauerstoffgehalt, der pH-Wert und die Temperatur des Inhalts in den einzelnen Wells bestimmt werden. In jedem der Wells ist z.B. ein Sauerstoffsensor vorgesehen, mit dem über Fluoreszenz der Sauerstoffgehalt der Flüssigkeit in dem jeweiligen Wells bestimmt wird. Die Mikrotiterplatte kann auf eine Optikplatte aufgesetzt werden. Die LED der Optikplatte beleuchtet den Sensor mittels eines divergierenden Strahlenbündels. Mit der Photodiode kann die Antwort des Sensors in dem Well ausgelesen werden. Mit der Vorrichtung ist es nicht möglich das Wachstum von Zellen, die Zelldichte, die Trübung etc. zu bestimmen.

Mikrotiterplatten, die eine Matrix aus mehreren starr miteinander verbundenen Behältnissen darstellen, sind hinlänglich aus dem Stand der Technik bekannt (siehe www.sigma-aldrich.com).

In vielfältigen Untersuchungen in diversen Bereichen, beispielsweise der Chemie, Pharmazie oder den Lebenswissenschaften, werden Proben in Behältnissen analysiert, welche zur Unterstützung der in einer jeweiligen Untersuchung interessierenden Prozesse bewegt werden. Dies geschieht bevorzugt mechanisch; dem Fachmann sind entsprechende Geräte wie beispielsweise Schüttler, Shaker oder Rocker bekannt. Solche Geräte sind kommerziell in Ausführungsformen erhältlich, welche ein Behältnis, aber auch eine Vielzahl von Behältnissen simultan in definierter Weise bewegen können. Zweck der Bewegung ist meist eine Durchmischung der Probe, welche als fluides Medium oder Flüssigkeit, beispielsweise Lösung, Emulsion oder Suspension vorliegt; die Probe kann auch ein fluides Medium sein, in welchem sich Mikroorganismen entwickeln.

Es ist daher Aufgabe der Erfindung, eine Vorrichtung zur Erfassung mindestens einer Veränderlichen mehrerer flüssiger Proben in mehreren Behältnissen während eines biologischen, chemischen oder biochemischen Prozesses zu schaffen, die eine unterbrechungsfreie, nicht-invasive und simultane Messung unter reproduzierbaren Bedingungen über längere Zeit an mehreren Behältnissen zulässt.

Diese Aufgabe wird gelöst durch eine Vorrichtung zur Erfassung mindestens einer Veränderlichen von flüssigen Proben in mehreren Behältnissen während eines biologischen, chemischen oder biochemischen Prozesses, die die Merkmale des Anspruchs 1 umfasst. Ebenso ist es Aufgabe der Erfindung ein Verfahren zur parallelisierten Erfassung mindestens einer Veränderlichen mehrerer flüssiger Proben in mehreren Behältnissen während eines biologischen, chemischen oder biochemischen Prozesses zu schaffen, das eine unterbrechungsfreie, nicht-invasive und simultane Messung unter reproduzierbaren Bedingungen über längere Zeit an mehreren Behältnissen mit einer flüssigen Probe zulässt.

Diese Aufgabe wird gelöst durch ein Verfahren zur Erfassung mindestens einer Veränderlichen von mehreren flüssigen Proben in mehreren Behältnissen während eines biologischen, chemischen oder biochemischen Prozesses, das die Merkmale des Anspruchs 6 umfasst. Ferner ist es Aufgabe der Erfindung ein System zur Erfassung mindestens einer Veränderlichen mehrerer flüssiger Proben in mehreren Behältnissen während eines biologischen, chemischen oder biochemischen Prozesses zu schaffen, das eine unterbrechungsfreie, nicht-invasive und simultane Messung unter reproduzierbaren Bedingungen über längere Zeit an einer Vielzahl von Behältnissen zulässt.

Diese Aufgabe wird gelöst durch ein System zur Erfassung mindestens einer Veränderlichen von mehreren flüssigen Proben in mehreren Behältnissen während eines biologischen, chemischen oder biochemischen Prozesses, das die Merkmale des Anspruchs 11 umfasst.

Die erfindungsgemäße Vorrichtung dient zur parallelisierten Erfassung mindestens einer Veränderlichen von mehreren Proben in mehreren Behältnissen während eines biologischen, chemischen oder biochemischen Prozesses. Die Veränderlichen sind im Allgemeinen die Trübung und die optische Dichte flüssiger Proben, sowie im Speziellen die Zelldichte, Biomasse- und Zellkonzentration, pH-Wert, O₂-Sättigung der Flüssigkeit und die Umgebungstemperatur.

Hierzu besitzt die erfindungsgemäße Vorrichtung einen Messträger in bzw. an dem mehrere Messeinheiten ortsfest angeordnet sind. Jede Messeinheit umfasst mindestens eine steuerbare Strahlungsquelle für elektromagnetische Strahlung und mindestens einen Sensor zur Detektion elektromagnetischer Strahlung. Der Messträger ist in X-Koordinatenrichtung und in Y-Koordinatenrichtung derart beweglich ausgebildet, dass der Messträger eine zusammengesetzte Bewegung in der X/Y-Ebene ausführt. Die Behältnisse sind in Form einer Matrix angeordnet. Die Matrix ist durch die mehreren Behältnisse definiert, die jeweils für sich eine quadratische Querschnittsform besitzen. Jedes der Behältnisse definiert eine umlaufende Wand und einen Boden. Der Boden verschließt das jeweilige Behältnis. Wie von einer Matrix bekannt sind die mehreren Behältnisse in Spalten und Zeilen angeordnet und folglich alle starr miteinander verbunden. Mikrotiterplatten sind derart ausgebildet, dass sie die Behältnisse mit der quadratischen Querschnittsform umfassen, die zur Aufnahme jeweils einer flüssigen Probe ausgestaltet sind. Die Matrix wird für die Messung lösbar aber ortsfest mit dem Messträger verbunden.

Die Behältnisse sind in der Matrix derart angeordnet, dass jedes Behältnis von vier Wänden umschlossen ist, die paarweise unter einen Winkel von 90° angeordnet sind. Zur Erfassung der Messdaten ist jedem Behältnis der Matrix am Boden eine Messeinheit zugeordnet, wenn die Matrix mit dem Messträger lösbar aber ortsfest verbunden ist. Ortsfest im Sinne der Erfindung bedeutet, dass jeweils eine Messeinheit jeweils einem Boden eines Behältnisses der Matrix zugeordnet ist, wenn die Matrix auf dem Messträger befestigt ist. Die Befestigung ist auch während der Schüttelbewegung des Messträgers nicht lösbar. Jede Messeinheit umfasst eine steuerbare Strahlungsquelle für elektromagnetische Strahlung und mindestens einen Sensor zur Detektion der aus dem Behältnis austretenden elektromagnetischen Strahlung. Gemäß der Erfindung ist ein Messbereich für die dem jeweiligen Behältnis zugeordnete Messeinheit im Bereich einer Ecke am Boden eines jeden Behältnisses ausgebildet. Im Bereich der Ecke des Messbereichs wird durch die Schüttelbewegung eine für die Messung durch die Messeinheit ausreichend große Flüssigkeitssäule ausgebildet. Der Boden eines jeden Behältnisses kann zumindest in einem Teilbereich oder ganz für die in das Behältnis eintretende und aus dem Behältnis austretende elektromagnetische Strahlung transparent sein.

Die durch den mindestens einen Sensor erfasste elektromagnetische Strahlung ist eine gestreute elektromagnetische Strahlung, die durch den Sensor kontinuierlich aufgezeichnet wird. Das Streulicht kann bei diesem Vorgang durch physikalische Vorgänge, wie z.B. Reflexion an Grenzflächen und Beugung, abhängig vom Brechungsindex der in der Probe vorliegenden Materie, erzeugt werden. Die gestreute elektromagnetische Strahlung entsteht somit auch durch die Streuung am im jeweiligen Behältnis vorhandenen biologischen Material. Bei der elektromagnetischen Strahlung kann es sich gemäß einer möglichen Ausführungsform um Licht mit einer Wellenlänge zwischen 600-900 nm handeln.

Mit der erfindungsgemäßen Vorrichtung können automatisiert und zeitlich parallel sich verändernde Prozessgrößen von Zellsuspensionen in kontinuierlich und nichtkontinuierlich geschüttelten Behältnissen erfasst werden. Zur Erzeugung einer Bewegung der Messträger für die Behältnisse kann eine in X-Koordinatenrichtung und in Y-Koordinatenrichtung zusammengesetzte Bewegung vorgesehen sein. Der Messträger kann dadurch mit einer definierten, radialen und orthogonal zur Schwerkraft verlaufenden Bewegung um eine feste Achse beweglich sein.

Die erfindungsgemäße Vorrichtung hat den Vorteil, dass es möglich ist, Veränderungen der Trübung sowie sich verändernde Zell- und Biomassenkonzentrationen in den Behältnissen infolge von Zellproliferationsvorgängen lebender Kulturen über die gesamte Kultivierungszeit zu verfolgen und aufzuzeichnen. Dabei können zeitlich parallel unterschiedliche Proben in unterschiedlichen Inkubationsumgebungen und deren Reaktion auf Reagenzien untersucht werden. Hinzu kommt, dass für eine Messung der Zell- und Biomassenkonzentrationen, die Behältnisse nicht zu einer Messapparatur transportiert werden müssen, die z.B. an einem anderen Ort vorgesehen ist. Die Messung kann gemäß der Erfindung automatisiert und zu beliebigen Zeitpunkten durchgeführt werden, ohne dass Bedienpersonal erforderlich ist.

Die Behältnisse der Matrix sind jeweils gegenüber dem Boden offen. Durch die Öffnung kann jedes Behältnis der Matrix individuell mit der entsprechenden Probe befüllt werden. Nach dem Befüllen kann die Matrix aus den Behältnissen, mit einer Abdeckung versehen werden. Die elektromagnetische Strahlungsquelle ist in der Messeinheit derart angeordnet, dass ein von der Strahlungsquelle ausgehender Strahl mit der Orthogonalen zur Bodenfläche eines jeden Behältnisses in einem Winkel zwischen 30° bis 45° angeordnet ist. Bevorzugter Weise beträgt der Winkel zwischen 36° - 42°. Besonders bevorzugt beträgt der Winkel 39°.

Der Sensor ist in der Messeinheit derart angeordnet, dass eine optische Faser des Sensors oder eine optische Achse des Sensors bei jedem Behältnis des Messträgers derart angeordnet ist, dass die optische Faser bzw. die optische Achse mit einer Orthogonalen zur Bodenfläche des jeweiligen Behältnisses unter einem Winkel 25° bis 30° angeordnet ist. Besonders bevorzugt beträgt der Winkel beträgt 27°.

Bevorzugter Weise besteht die Strahlungsquelle aus mindestens einer Leuchtdiode, der ein optisches System zur Lenkung und Formung (Kollimation) der elektromagnetischen Strahlung mit einer Wellenlänge von 600-900 nm nachgeordnet ist. Das Licht der Leuchtdiode wird durch das optische System in das jeweils zugeordnete Behältnis eingestrahlt. Im Wesentlichen wobei die Linse die elektromagnetische Strahlung in der flüssigen Probe zu einem Zylinder kollimiert.

Gemäß einer möglichen Ausführungsform, ist der mindestens eine Sensor mit einer optischen Faser und mindestens einem optischen Filter gekoppelt. Die Strahlungsquelle und die optische Faser des Sensors sind in der Messeinheit unter einem Winkel zueinander angeordnet. Die Strahlungsquelle und optische Faser sind bevorzugt in einem definierten Winkel von 90° zueinander angeordnet.

Bevorzugt ist jeder Messträger mit einem Elektronikmodul versehen, mit dem auch eine Datenverbindung (z.B. eine Funkverbindung) zu einer Basisstation besteht. Das Elektronikmodul dient auch zur Energieversorgung der Leuchtdiode und ggf. der Sensoren, zur Ansteuerung der Leuchtdiode einer jeden Messeinheit eines jeden Behältnisses und der Messwertaufnahme mittels dem Sensor der Messeinheit.

Durch eine zylindrische Leuchtdiode mit maximal 3 mm Durchmesser und wenigstens 7000 mCd Strahlungsleistung wird in Kombination mit dem nachgeordneten optischen System (Kollimator), elektromagnetische Strahlung einer Dominanzwellenlänge im Bereich zwischen 600-900 nm durch den für die Strahlung durchlässigen Boden oder zumindest durchlässigen Messbereich in das jeweilige Behältnis (Kleinstbioreaktor der Matrix) eingestrahlt. Der Strahl ist derart kollimiert, dass in der flüssigen Probe ein Lichtzylinder entsteht, der maximal 1,5 mm im Durchmesser auf einer Länge von wenigstens 10 mm vom Austrittsort entfernt, aufweist. Das an der Probe erzeugte Streulicht wird über mindestens eine Lichtfaser dem Sensor der Messeinheit durch einen optischen Filter zugeführt. Als Sensoren bzw. Lichtsensoren können beispielsweise für eine Kopplung mit Lichtfasern geeignete Low-Noise Si-Photodioden mit integriertem Verstärker mit einer Messfrequenz von wenigstens 10 kHz zum Einsatz kommen.

Eine mögliche Form der Matrix aus den Behältnissen kann sein, dass jedes der Behältnisse ein quadratischer Kleinstbioreaktor mit transparentem Boden ist, der sich über dem Sensor der Messeinheit des Messträgers zur Erfassung von Trübung, Biomassenkonzentration und Zellkonzentration befindet. Mehrere der quadratischen Kleinstbioreaktoren sind in der Matrix angeordnet. Die Matrix wird durch einen Spritzgussprozess hergestellt, so dass alle Behältnisse der Matrix starr miteinander verbunden sind. Die Matrix wird kontinuierlich um eine feste Achse mit einem Radius von 1-30 mm und einer Frequenz von 0-600 Upm bewegt. Dadurch wird die Probe in Richtung der Wände (Seitenwände) des Behältnisses (Kleinstbioreaktor) zentrifugiert und es bildet sich in den Ecken eine Flüssigkeitssäule aus, so dass im Messbereich ausreichend Messsignale erzeugt werden können.

Das erfindungsgemäße Verfahren dient zur Bestimmung mindestens einer Veränderlichen von mehreren Proben in mehreren Behältnissen eines biologischen, chemischen oder biochemischen Prozesses einer sich in mindestens einem bewegten Behältnis einer Matrix befindlichen, flüssigen Probe. Die wesentlichen Aspekte des erfindungsgemäßen Verfahrens sind das Bewegen der mehreren, in Form einer Matrix angeordneten und starr miteinander verbundenen Behältnisse, in definierter Weise und das Bestimmen der mindestens einen Veränderlichen der flüssigen Probe, wobei die Bestimmung mit einem durch die Bewegung des Behältnisses sich ergebenden Signalmusters der einzelnen flüssigen Proben zeitlich abgestimmt wird. Vor der eigentlichen Messung erfolgt das Befüllen von mindestens einem der mehreren in Form einer Matrix angeordneten Behältnisse, wobei die Matrix ortsfest und definiert auf einem Messträger angeordnet ist. Beim Bewegen des Messträgers kann dabei mindestens eine Veränderliche während des biologischen, chemischen oder biochemischen Prozesses bestimmt werden.

Die Bewegung des Messträgers wird ununterbrochen und mit einer definierten, radialen und orthogonal zur Schwerkraft verlaufenden Bewegung (zusammengesetzt aus einer X-Koordinatenrichtung und einer Y-Koordinatenrichtung) um eine feste Achse durchgeführt. In jedem Behältnis, in dem sich eine Probe befindet, wird mit der dem Behältnis fest zugeordneten Messeinheit des Messträgers die Veränderliche der Probe bestimmt. Die Messeinheiten sind im Messträger derart angeordnet, dass bei der auf den Messträger aufgesetzten Matrix je eine Messeinheit je einem Behältnis zugeordnet ist. Die Messeinheit besteht aus einer steuerbaren Strahlungsquelle und mindestens einem Sensor, mit dem die mindestens eine Veränderliche der Probe in dem jeweiligen Behältnis lokalisiert erfasst wird.

Zur Erfassung der mindestens einen Veränderlichen der Probe, wird von der Strahlungsquelle durch den Boden des jeweiligen Behältnisses eine elektromagnetische Strahlung in die Probe eingestrahlt. Durch den Boden wird dann von mindestens einem Sensor der Messeinheit die gestreute elektromagnetische Strahlung der mindestens einen Veränderlichen der Probe empfangen. Mittels des Sensors kann eine kontinuierliche, optische Messung und Aufzeichnung von Streulicht, das am biologischen Material infolge der Bestrahlung mit elektromagnetischer Strahlung entsteht, durchgeführt werden. Die mindestens eine Veränderliche kann anhand der an suspendierten Partikeln oder an biologischem Material gestreuten, elektromagnetischen Strahlung ermittelt werden. Diese optische Antwort wird von dem Sensor erfasst, der dem jeweiligen Behältnis ortsfest zugeordnet ist.

Bei einer Veränderlichen kann es sich um den pH-Wert handeln. Bei einer weiteren Veränderlichen kann es sich um die relative Sättigung an gelöstem Sauerstoff in der Probe handeln, die ebenfalls als optische Antwort von dem Sensor, der dem jeweiligen Behältnis zugeordneten ist, erfasst werden kann. Die relative Sättigung an gelöstem Sauerstoff in der jeweiligen flüssigen Probe kann durch eine Veränderung des Energieeintrags bei der Bewegung der Behältnisse bzw. des Trägers geregelt werden.

Zur Messung von pH-Wert und der Gelöstsauerstoffkonzentration in den Behältnissen (Kleinstbioreaktoren) werden Chemosensoren verwendet, die am Boden der Kleinstbioreaktoren befestigt sind. Die Chemosensoren enthalten Photolumineszenz-Farbstoffe, die bei Bestrahlung mit elektromagnetischer Strahlung einer spezifischen Wellenlänge elektromagnetische Strahlung mit spezifischer Wellenlänge in definierter Weise abgeben. Die Chemosensoren reagieren bei direktem Kontakt mit der in den Kleinstbioreaktoren vorliegenden Probe mit Änderung der Intensität und Abklingzeit der Lumineszenz, die sich nach Messung durch einen Lichtsensor mittels mathematischer Verfahren in die Größen wie z.B. pH-Wert und Konzentration des gelösten Sauerstoffes überführen lassen.

Bevorzugter Weise umfasst die Strahlungsquelle mindestens eine Leuchtdiode, der ein optisches System zur Lenkung der elektromagnetischen Strahlung zugeordnet wird. Bei der elektromagnetischen Strahlung handelt es sich um Licht mit einer Wellenlänge von 600-900 nm. Die mindestens eine Veränderliche kann unabhängig von der geometrischen Dimension einer radialen Auslenkung der Bewegung erfasst werden. Das Messverfahren nimmt durch den Sensor die Messwerte mit einer hohen Messfrequenz auf, die es erlaubt 5000 bis 100.000 Einzelmesswerte während der periodisch schwankenden Höhe der Flüssigkeitssäule über dem ortsfesten Sensor zu generieren.

Für eine hochaufgelöste Erfassung der sich periodisch verändernden Höhe des Streulichtsignals ist eine hohe Messfrequenz des Lichtsensors notwendig. Diese kann im höheren kHz oder MHz Bereich liegen, um anhand periodisch wiederkehrender, konstanter Bereiche innerhalb des Messsignals Messdaten zur Korrelation mit Referenzgrößen (OD₆₀₀, Biotrockenmasse, Zellkonzentration) zu extrahieren.

Das vom Sensor der Messeinheit gemessene Streulichtsignal wird zur Umrechnung in Referenzgrößen wie optische Dichte, Biomasse oder Zellkonzentration zu einem festen Zeitpunkt (in der Regel nach Beginn des Prozesses oder der Reaktion) aus einem definierten Intervall der Rohdaten mittels geeigneter mathematischer Verfahren berechnet. Die Berechnung wird in einer Basisstation (wie z.B. einem Computer) durchgeführt, mit der jeder Messträger über eine Datenverbindung verbunden ist. Die Kommunikation zwischen Träger und Basisstation erfolgt in bevorzugter Weise drahtlos.

Ein mathematisches Auswerteverfahren läuft auf der Basisstation, wobei der erhaltene zeitaufgelöste Streulichtsignalverlauf mit wenigsten 10.000 Messereignissen pro Sekunde in einen einzelnen, zu einem festen Zeitpunkt nach Prozessbeginn erfassten Messwert, überführt wird. Das mathematische Auswerteverfahren ist derart konzipiert, dass Messdaten periodisch wiederkehrender Bereiche innerhalb definierter Bereiche, die im Voraus definierte Kriterien erfüllen, selektiert und weiterverarbeitet werden.

Mit dem erfindungsgemäßen System besteht in erster Linie die Möglichkeit einer simultanen Überwachung veränderlicher Prozessgrößen von hochgradig parallelisierten biologischen und (bio-)chemischen Prozessen. Dies ist z.B. mit bis zu 240 Behältnissen (wie z.B. Kleinstbioreaktoren) mit einem Arbeitsvolumen von 500-11000 µl innerhalb einer Inkubations-/Schüttlerumgebung möglich. Die starke Miniaturisierung der Messträger jeweils mindestens einer Mikrotiterplatte (Matrix aus 24 Behältnissen) ermöglicht die Anwendung mindestens eines Messträgers pro Inkubationsumgebung und/oder Schüttlerumgebung. Mehrere zusammengefasste Messträger können in unterschiedlichen Inkubations- und/oder Schüttlerumgebungen eingesetzt werden und ermöglichen somit die Variation technischer Versuchsparameter wie Temperatur, O₂/CO₂-Sättigung der Umgebungsluft und Schüttelfrequenz.

Das erfindungsgemäße System zur parallelisierten Erfassung mindestens einer Veränderlichen in mehreren Behältnissen für flüssige Proben während eines biologischen, chemischen oder biochemischen Prozesses umfasst mehrere Behältnisse (Kleinstbioreaktoren mit quadratischen Querschnitt) zur Aufnahme einer flüssigen Probe. Die Behältnisse sind zu einer Matrix zusammenfasst. Die Behältnisse sind somit durch die Matrix in mehreren Spalten und Zeilen auf mindestens einem Messträger ortsfest positioniert. Der Messträger selbst besitzt eine Vielzahl von Messeinheiten. Jedem Behältnis der Matrix, die auf dem Messträger positioniert und ortsfest mit diesem verbunden ist, ist am Boden jeweils eine Messeinheit zugeordnet. Die Messeinheit umfasst eine steuerbare Strahlungsquelle für elektromagnetische Strahlung und mindestens einen Sensor zur Detektion der elektromagnetischen Strahlung. Jeder Messträger verfügt über ein Elektronikmodul bzw. ein Elektronikmodul ist auf dem Messträger angeordnet, das zur Steuerung der Strahlungsquelle und zur Detektion der elektromagnetischen Strahlung durch den Sensor dient. Eine Bewegungseinrichtung sorgt dafür, dass der mindestens eine Messträger mit einer definierten, radialen und orthogonal zur Schwerkraft verlaufenden Bewegung um eine feste Achse beweglich ist.

Gemäß einer möglichen Weiterbildung der Erfindung kann die Bewegungseinrichtung in einen Inkubator zusammen mit dem mindestens einen Messträger aufgenommen werden.

Eine Basisstation kann über das Elektronikmodul mit dem mindestens einen Messträger im Inkubator kommunizieren. Die Kommunikation der Basisstation mit dem mindestens einen Messträger im Inkubator kann über eine Datenverbindung (z. B. eine Funkverbindung (Bluetooth, WLAN)) bereitgestellt werden. Die starke Miniaturisierung der Messträger für Mikrotiterplatten aus jeweils vierundzwanzig Behältnissen (Kleinstbioreaktoren) ermöglicht die Anwendung mindestens eines Messträgers pro Inkubationsumgebung. Mehrere Messträger (bis zu zehn Messträger) können auf der Bewegungseinrichtung im Inkubator untergebracht werden. Dadurch ergibt sich in vorteilhafter Weise eine unterbrechungsfreie, nicht-invasive und simultane Messung an mehreren Behältnissen, auch auf mehreren Trägern.

Die einzelnen Behältnisse haben einen quadratischen Querschnitt und ein Volumen von 500 bis 11000 µl das zur Aufnahme der flüssigen Probe dient. Mehrere Messträger können in mehreren unterschiedlichen Inkubatoren positioniert sein, so dass die Messträger unterschiedlichen Inkubationsumgebungen und Bewegungen der Bewegungseinrichtung unterliegen. Dies hat den Vorteil, dass man unterschiedliche Inkubations- und/oder Schüttlerumgebungen für gleiche Proben realisieren kann, damit auf einfache Weise eine Variation der technischen Versuchsparameter, wie z. B. Temperatur, O₂/CO₂-Sättigung der Umgebungsluft und Schüttelfrequenz realisiert werden können.

Die Messträger in den mehreren Inkubatoren sind in bevorzugter Weise drahtlos (wie z. B. WLAN, Bluetooth) mit der Basisstation verbunden. In einer weiteren Ausführungsform werden Kombinationen von draht-gebundener und drahtungebundener Kommunikation für die Erfindung festgelegt.

Die Messeinheiten, die sich auf einer Schüttlerplattform innerhalb einer Inkubationsumgebung befinden, und die Kommunikationsstruktur, zwischen den einzelnen Messeinheiten und einer Basisstation zur Datenverarbeitung / Datenaufzeichnung, benötigen eine elektrische Vorrichtung zum kabelgebundenen oder drahtlosen Datentransfer. Jede Messeinheit verfügt beispielweise über einen Funktransmitter/-receiver mit dem ein lokales Funknetzwerk zu einem fest stationierten, zentralen Funktransmitter/-receiver hergestellt wird. Bei der verwendeten Datentransfertechnologie können beispielsweise Bluetooth oder WLAN genutzt werden. Alle Messeinheiten bzw. deren Elektronikeinheiten verfügen in einer möglichen Ausführungsform über einen geräteinternen dauerhaften Datenspeicher zur Aufzeichnung von Messdaten. Der zentrale Funktransmitter/-receiver ist über eine Datenschnittstelle mit einem Gerät zur Datenverarbeitung / Datenaufzeichnung wie beispielsweise einem Rechner verbunden. Rechner umfassen Desktop-Rechner, Notebook-Computer, Tablet-Computer oder Smart-Phones.

Die Erfindung ermöglicht eine Vorrichtung, ein Verfahren und ein System zur automatisierten und parallelisierten Erfassung mindestens einer veränderlichen Prozessgröße von Zellsuspensionen in kontinuierlich und nicht kontinuierlich geschüttelten, quadratischen Behältnissen (Kleinstbioreaktoren), die eine Matrix definieren. Eine kontinuierliche Überwachung kritischer Prozessparameter im Bereich der kommerziellen Biotechnologie und F&E ist zur Beurteilung von Wachstumsprozessen bzw. der Teilungsfähigkeit von Zellkulturen (Prokaryoten und Eukaryoten) notwendig. Diese messbare Eigenschaft von Zellen wird genutzt um optimale Kultivierungsbedingungen, vorteilhafte Nährstoffe und Substrate, vorteilhafte Zellstämme und genetische Varianten, Wachstumsinhibitoren und Toxine aus einer Vielzahl von Variationen zu ermitteln.

Die Erfindung hat den Vorteil, dass mögliche Veränderungen der Trübung sowie sich verändernde Zell- und Biomassenkonzentrationen infolge Zellproliferationsvorgängen lebender Kulturen über die gesamte Kultivierungszeit verfolgt und aufgezeichnet werden können. Das zugrundeliegende Messprinzip basiert auf dem Einstrahlen elektromagnetischer Wellen in die in den Behältnissen vorliegenden Zellsuspensionen, wobei jedes Behältnis eine relativ zum Behältnis unbewegliche Strahlungsquelle aufweist, der ein korrespondierender Lichtsensor zugeordnet ist. Alle Messvorgänge können im kontinuierlichen sowie nicht-kontinuierlichen Schüttelbetrieb ausgelöst und aufgezeichnet werden.

Anwendungsgebiete der Erfindung bestehen in erster Linie in der simultanen Überwachung veränderlicher Prozessgrößen hochgradig parallelisierter biologischer und (bio-)chemischer Prozesse mit bis zu 240 Behältnissen ä 500-11000 µl innerhalb einer Inkubations-/Schüttlerumgebung. Die starke Miniaturisierung der Messträger für jeweils vierundzwanzig Behältnisse ermöglicht die Anwendung mindestens eines Messträgers pro Inkubationsumgebung.

Die Vielzahl der zu einer Matrix zusammenfassten Behältnisse, als Mikrotiterplatte bezeichnet, wird aus Kunststoff mit einem Spritzguss- oder Abformverfahren hergestellt. Der Kunststoff kann z. B. Polycarbonat oder Polystyrol sein.

Erfasste Prozessgrößen sind im Allgemeinen Trübung und optische Dichte sowie im Speziellen Zelldichte, Biomasse- und Zellkonzentration, pH-Wert, O₂-Sättigung der Flüssigkeit und die Umgebungstemperatur.

Die Integration möglichst vieler Messträger (Zellkultur-Inkubatoren) wird durch eine hohe Miniaturisierung der Messträger und Messeinheiten erreicht.

Als Anwendungsgebiete kommen vor allem Screenings (Stammselektion, genetische Selektionen, Toxizitätstests) und Operationen der Bioprozessentwicklung (Medienoptimierung, Substratselektion, Optimierung des O₂-Eintrages) in denen biologische oder chemische Veränderungen der Trübung von Suspensionen überwacht und Reaktionsparameter in derselben Inkubationsumgebung miteinander verglichen werden sollen in Betracht.

Die Erfindung und ihre Vorteile werden nachfolgend unter Rückgriff auf die beigefügten Zeichnungen näher beschrieben.
**Figur 1A** zeigt eine schematische Seitenansicht eines Behältnisses mit quadratischen Querschnitt für eine Probe mit der zugeordneten Messeinheit.
**Figur 1B** zeigt eine schematische Seitenansicht eines Behältnisses für eine Probe mit der zugeordneten Messeinheit, wobei die Ansicht im Vergleich zu Fig. 1A um 90° um die Achse gedreht ist.
**Figur 1C** zeigt eine Draufsicht auf das Behältnis mit quadratischen Querschnitt und der zugeordneten Messeinheit.
**Figur 2** zeigt eine Draufsicht auf ein Behältnis mit quadratischer Querschnittsform, wobei in einer Ecke der Messbereich dargestellt ist.
**Figuren 3A** - **3B** zeigen jeweils einen Querschnitt durch das optische System zur Kollimation der durch die Strahlungsquelle abgegebenen elektromagnetischen Strahlung.
**Figur 4** zeigt eine Draufsicht auf eine Anordnung aus einer Matrix aus mehreren Behältnissen auf einem Messträger.
**Figur 5A** zeigt eine Draufsicht auf eine Anordnung einer Matrix aus mehreren Behältnissen, die in starrer Verbindung mit dem Messträger sind.
**Figur 5B** zeigt eine Draufsicht auf eine Anordnung der Matrix aus mehreren Behältnissen auf dem Messträger, wobei die Behältnisse in Gruppen eingeteilt sind.
**Figur 5C** zeigt eine Seitenansicht der Anordnung der Matrix aus mehreren Behältnissen, die mit einer Ausführungsform des Messträgers starr verbunden ist.
**Figur 6A** zeigt eine Draufsicht auf eine Anordnung der Matrix aus mehreren Behältnissen 1 in starrer Verbindung zueinander auf einer anderen Ausführungsform des Messträgers.
**Figur 6B** zeigt eine Schnittansicht entlang der in Figur 6A gekennzeichneten Schnittlinie des Messträgers für die Matrix aus den Behältnissen, wobei der Messträger auf einer Bewegungseinrichtung platziert ist.
**Figur 7A** zeigt eine graphische Darstellung eines typischen Streulichtsignals einer Zellsuspension konstanter optischer Dichte über ein definiertes Zeitintervall, das durch periodisch wiederkehrende Blöcke charakterisiert ist.
**Figur 7B** zeigt eine graphische Darstellung der Größen-Sortierung von Streulichtsignaldaten aus einem definierten Zeitintervall aus Figur 7A.
**Figur 7C** zeigt eine graphische Darstellung der Ableitung des sortierten Streulichtsignalverlaufs aus Figur 7B.
**Figur 7D** zeigt eine graphische Darstellung des Messrauschens aus Figur 7A.
**Figur 8** zeigt die Anordnung von mehreren Mikrotiterplatten mit den quadratischen Behältnissen in einem Inkubator.
**Figur 9** zeigt eine schematische Anordnung eines Inkubators zu einem Computer, der für die Aufnahme und Auswertung der Messergebnisse der Substanzen in den Behältnissen der Mikrotiterplatten sorgt, die im Inkubator eingebracht sind.
**Figur 10** zeigt ein Ablaufdiagramm einer Ausführungsform des erfindungsgemäßen Verfahrens.

Die Zeichnungen zeigen lediglich Ausführungsformen, wie das bzw. die erfindungsgemäßen Behältnisse oder die erfindungsgemäße Vorrichtung ausgestaltet sein können. Die Zeichnungen stellen ausdrücklich keine Beschränkung der Erfindung auf diese Ausführungsformen dar.

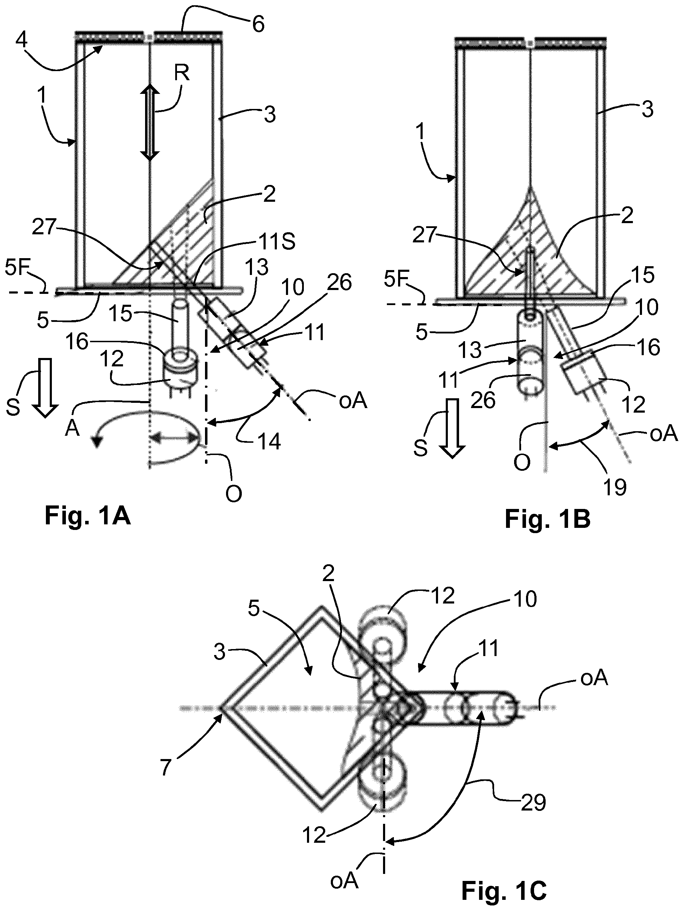

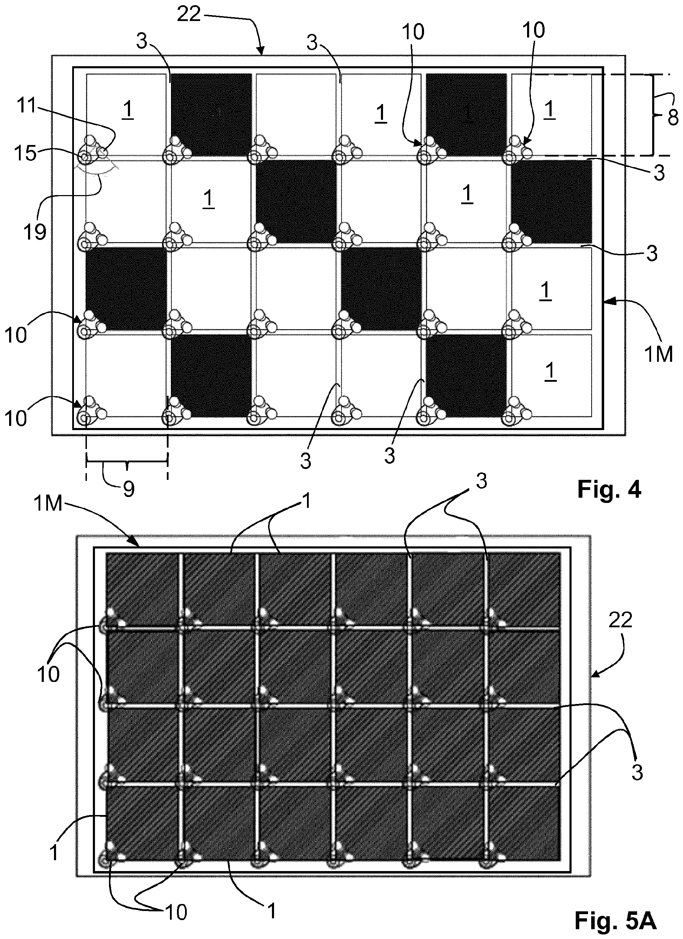

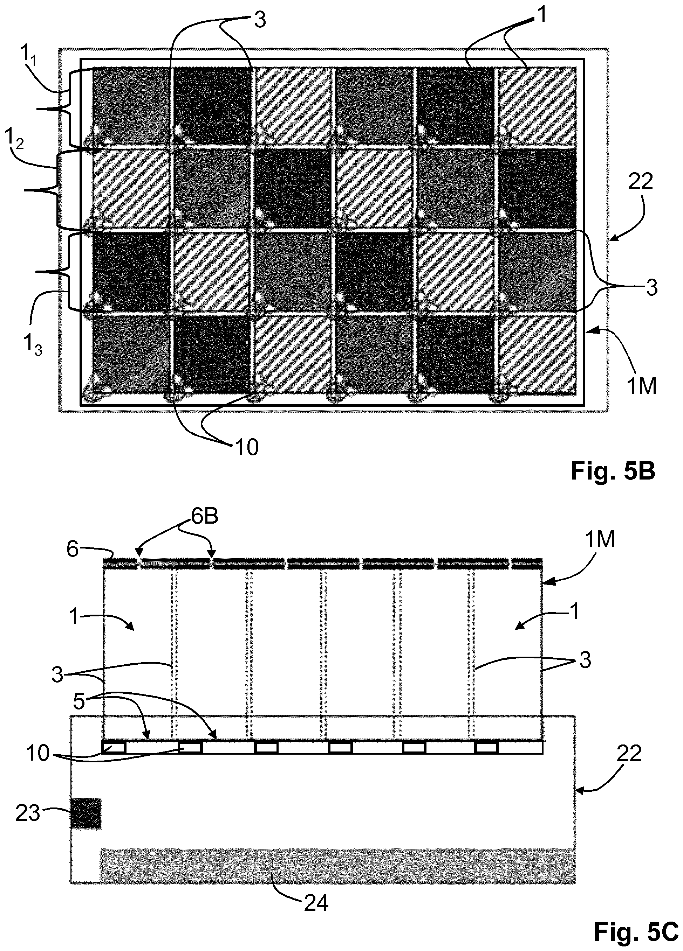

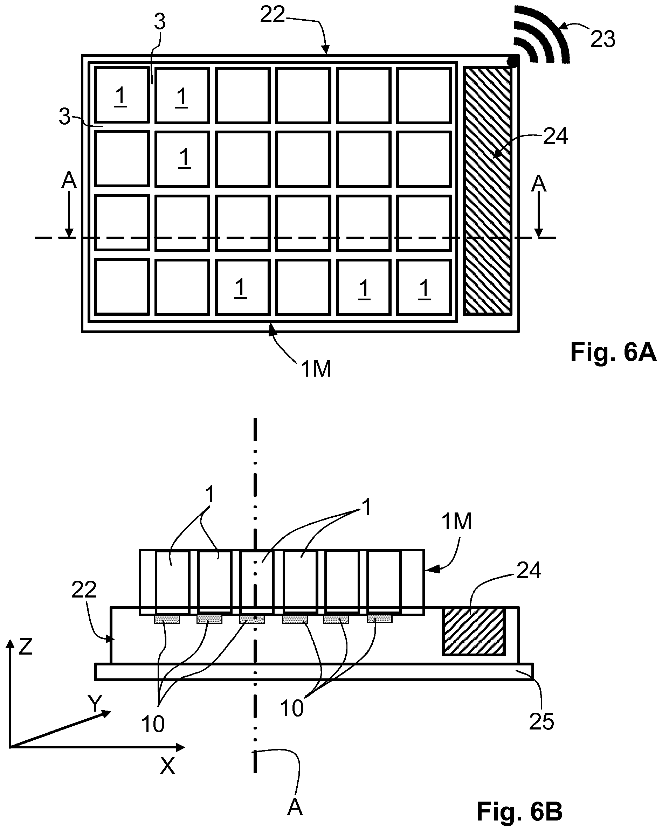

**Figur 1A** zeigt eine schematische Seitenansicht der Ausgestaltung eines Behältnisses 1, wie es in einer Matrix 1M (siehe Figur 5) ausgebildet ist, für eine Probe 2 mit einer dem Behältnis 1 zugeordneten Messeinheit 10. Gemäß einer möglichen Ausführungsform können mehrere dieser Behältnisse 1 zu der Matrix 1M zusammengefasst werden, wobei die Matrix 1 M ortsfest und starr auf einem Messträger 22 (siehe Figur 5C oder 6B) angebracht werden kann. Als Probe 2 ist ein fluides Medium (Flüssigkeit), das beispielsweise in Form einer Lösung, einer Emulsion oder einer Suspension vorliegt, anzusehen. Die Probe 2 kann auch ein fluides Medium sein, in welchem sich Mikroorganismen entwickeln. Das Behältnis 1 ist durch eine umlaufende Wand 3 und einen Boden 5 definiert. Gegenüber zum Boden 5 besitzt das Behältnis 1 eine Öffnung 4, durch die die Probe 2 in das Behältnis 1 gefüllt werden kann. Die Öffnung 4 des Behältnisses 1 kann, falls erforderlich, mit einer Abdeckung 6 verschlossen werden. Der Boden 5 ist derart gestaltet, dass er für beide zum Boden 5 bzw. einer Bodenfläche 5F orthogonalen Richtung R für elektromagnetische Strahlung (Licht), die zur Beleuchtung und Detektion verwendet wird, durchlässig ist. Zumindest eine Messung an der Probe 2 dient zur Gewinnung von Information mittels einer optischen Methode, wobei die Bestimmung mindestens einer Veränderlichen (wie z. B. Trübung, Biomassen- oder Zellkonzentration) während einer ununterbrochenen, definierten, radialen, orthogonal zur Schwerkraft laufenden Bewegung der Behältnisse 1 um eine feste Achse A erfolgt. Der Radius der Bewegung kann zwischen 1 - 50 mm liegen. Die Frequenz der Bewegung kann zwischen 0 - 600 Umdrehungen pro Minute (Upm) liegen.

Zur Beleuchtung und Detektion ist jedem Boden 5, außerhalb eines jeden Behältnisses 1 der Matrix 1M jeweils eine Messeinheit 10 zugeordnet. Die Messeinheit 10 besteht aus mindestens einer steuerbaren Strahlungsquelle 11 und mindestens einem Sensor 12 zur Bestimmung der mindestens einen Veränderlichen der Probe 2 im Behältnis 1. Die Strahlungsquelle 11 besteht z. B. aus einer Leuchtdiode 26 der ein optisches System 13 zur Lenkung, Formung und Übertragung elektromagnetischer Strahlung mit definierter Wellenlänge zugeordnet ist. Der Strahl 11S wird durch das optische System 13 derart kollimiert, dass in der Probe 2 ein Lichtzylinder 27 entsteht.

Gemäß einer möglichen Ausführungsform beträgt die Wellenlänge 600-900 nm. Der Strahl 11S der Strahlungsquelle 11 wird in einem definierten Winkel 14 in das jeweils zugeordnete Behältnis 1 gelenkt. Der definierte Winkel 14 zwischen dem Strahl 11S bzw. der optischen Achse oA der Strahlungsquelle 11 und der Orthogonalen O zum Boden 5 beträgt zwischen 30° - 45°. Bevorzugter Weise beträgt der definierte Winkel 14 zwischen 36° - 42°. Der Sensor 12 ist an eine optische Faser 15 und mindestens einen optischen Filter 16 gekoppelt.

Wie in **Figur 1B** schematisch dargestellt ist, wird der Sensor 12 in einem definierten Winkel 19 in Bezug auf den Boden 5 des Behältnisses 1 angeordnet. Der Winkel 19 zwischen der Orthogonalen O zum Boden 5 und der optischen Faser 15 des Sensor 12 bzw. dessen optischen Achse oA wird zwischen 25° - 30° festgelegt. In bevorzugter Weise beträgt der Winkel 19 29°.

Das Behältnis 1 und folglich die gesamte Matrix 1M wird in definierter Weise bewegt. Die Bestimmung der mindestens einen Veränderlichen einer Probe 2 in einem Behältnis 1 erfolgt während einer ununterbrochenen, definierten, radialen, orthogonal zur Schwerkraft S laufenden Bewegung des Behältnisses 1 bzw. der Matrix 1M um eine feste Achse A.

**Figur 1B** zeigt eine schematische Seitenansicht eines Behältnisses 1 für eine Probe 2 mit der zugeordneten Messeinheit 10, wobei die Ansicht im Vergleich zu Fig. 1A um 90° um die Achse A gedreht ist.

**Figur 1C** zeigt beispielsweise eine Draufsicht auf ein Behältnis 1 der Matrix 1M mit der zugeordneten Messeinheit 10. Das Behältnis 1 hat einen quadratischen Querschnitt. Die hier beschriebene quadratische Querschnittsform stellt eine für die Erfindung wesentliche Querschnittsform dar. Unter dem Boden 5 des Behältnisses 1 ist die Messeinheit 10 dem Behältnis 1 zugeordnet. Die Messeinheit 10 ist in oder auf einem hier nicht dargestellten Messträger 22 angeordnet. Die Matrix 1M mit den Behältnissen 1 wird auf dem Messträger 22 gesetzt, so dass die mindestens eine Strahlungsquelle 11 und der mindestens eine Sensor 12 der Messeinheit 10 einem jeden Boden 5 der Behältnisse 1 der Matrix 1M zugeordnet sind.

Wie in **Figur 1C** dargestellt erreicht man durch die Schüttelbewegung der Matrix 1M aus den Behältnissen 1 stets eine Anhäufung der flüssigen Probe 2 in einer Ecke 7 (Zusammentreffen von zwei Wänden 3 unter einem bestimmten Winkel) des Behältnisses 1. Bei der hier dargestellten Ausführungsform ist die Messeinheit 10 derart im Messträger 22 ortsfest verbaut, dass die mindestens eine Strahlungsquelle 11 und der mindestens eine Sensor 12 in einem Messbereich 17 am Boden 5 eines jeden Behältnisses 1 lokalisiert ist. Der Messbereich 17 ist bei jedem Behältnis 1 in dem Randbereich des Behältnisses 1 angeordnet, an dem sich beim Schütteln des Behältnisses 1 die größte Ansammlung der flüssigen Probe 2 befindet.

Bei der hier dargestellten Ausführungsform umfasst die Messeinheit 10 eine Strahlungsquelle 11 und zwei Sensoren 12. Die optische Achse oA der Strahlungsquelle 11 und optische Achse oA des Sensors 12 sind in einem definierten Winkel 29 zueinander angeordnet. Bevorzugter Weise beträgt der Winkel 90°. Die Strahlungsquelle 11 und zwei Sensoren 12 sind in einer gemeinsamen Halterung (nicht dargestellt) angeordnet, welche die Messeinheit 10 darstellt. Diese Anordnung der Messeinheit 10 gewährleistet, dass auch bei kleinen Probenvolumina eine möglichst hohe Flüssigkeitssäule über dem mindestens einen Sensor 12 der Messeinheit 10 vorliegt, der für eine reproduzierbare Messung des Streulichtes der Probe 2 notwendig ist.

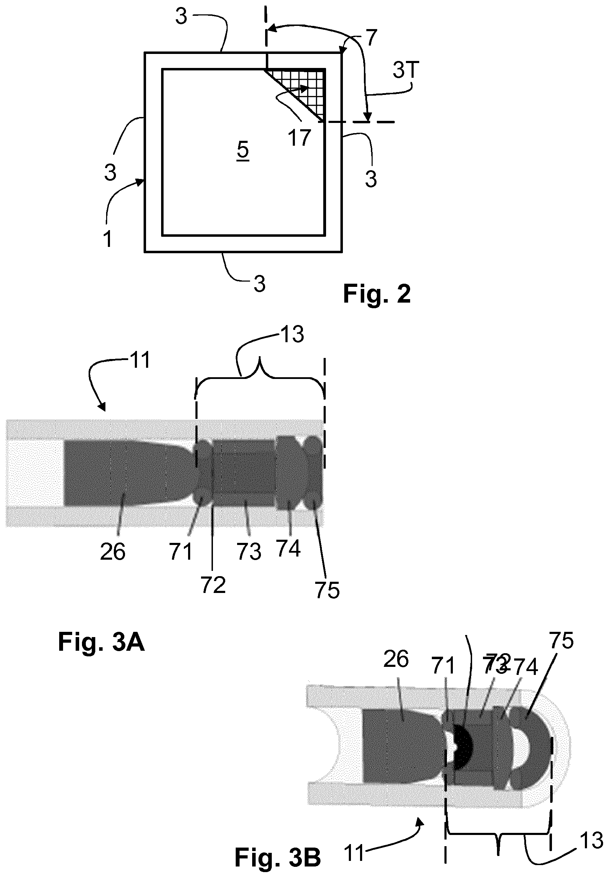

**Figur 2** zeigt beispielsweise eine Draufsicht auf ein Behältnis 1 der Matrix 1M für eine Probe 2. Das Behältnis 1 ist in dieser Ausführungsform durch vier Wände 3 und einen Boden definiert, so dass sich die quadratische Querschnittsform ergibt. Die quadratische Querschnittsform hat den Vorteil, dass es bei der Schüttelbewegung der Matrix 1M aus den Behältnissen 1 zu einem gleichmäßigen Aufbau der Flüssigkeitssäule der Probe 2 im Bereich der Ecken 7 der Behältnisse 1 kommt. Der Messbereich 17 ist folglich im Bereich der einen Ecke 7 vorgesehen. Jedes Behältnis 1 kann derart ausgestaltet sein, dass zumindest der Messbereich 17 am Boden 5 des Behältnisses 1 in beiden zum Boden 5 orthogonalen Richtungen für die elektromagnetische Strahlung durchlässig ist. Der Messbereich 17 eines jeden Behältnisses 1 ist im Randbereich ausgebildet, in dem sich bei der Schüttelbewegung die Flüssigkeitssäule der Probe 2 aufbaut. Diesem Messbereich 17 ist jeweils eine Messeinheit 10 ortsfest zugeordnet, wenn die Matrix 1M auf dem Messträger 22 (siehe Fig. 4) angebracht ist.

In der Figur 2 ist die wesentliche Ausführungsform der Behältnisse 1 mit quadratischem Querschnitt beschrieben. Außerhalb des Schutzumfangs der beanspruchten Erfindung ist es vorstellbar, dass mit dem Messträger 22 und seinen Messeinheiten 10 jede Art von eckigen Behältnissen 1 verwendet werden kann, so dass die Behältnisse 1 eine Vielzahl von

Ausführungen der Querschnittsform in eckiger Form annehmen können. Einzige Bedingung ist, dass sich bei einer Schüttelbewegung der mehreren in Form der Matrix 1M und starr miteinander verbundenen Behältnisse 1 in jedem der mit der Probe befüllten Behältnisse 1 im Messbereich 17 für die Messung durch die Messeinheit 10, eine entsprechende Flüssigkeitssäule aufbaut.

**Figuren 3A** - **3B** zeigen jeweils einen Querschnitt durch das optische System 13 zur Kollimation der durch die Strahlungsquelle 11 abgegebenen elektromagnetischen Strahlung. Die Strahlungsquelle 11 besteht in einer Ausführungsform aus der Leuchtdiode 26, auf die ein optisches System 13 folgt. Das optische System 13 umfasst einen Abstandshalter 71, eine Lochblende 72, einen weiteren Abstandshalter 73, eine optischen Linse 74 und einen Abstandshalter 75. Die Lochblende 72 dient der Verringerung des spezifischen Abstrahlwinkels der Leuchtdiode 26. Der aus der Lochblende 72 tretende Lichtkegel wird durch die optische Linse 74 fokussiert, wodurch eine hohe Tiefenschärfe der Projektion erreicht wird.

**Figur** 4 zeigt eine Draufsicht auf eine mögliche Ausführungsform einer Anordnung von einer Matrix 1M aus mehreren Behältnissen 1 auf einem Messträger 22. Wie in Figur 4 dargestellt ist, sind die mehreren Behältnisse 1 der Matrix 1M starr miteinander verbunden und durch die Wände 3 voneinander getrennt. Die Behältnisse 1 sind in der hier dargestellten Ausführungsform der Matrix 1M regelmäßig in Spalten 9 und Zeilen 8 angeordnet. Auf dem Messträger 22 sind die Messeinheiten 10 angeordnet bzw. integriert. Durch das Aufsetzen der Matrix 1M auf den Messträger 22, ist je eine Messeinheit 10 ortsfest je einem Behältnis 1 der Matrix 1M zugeordnet. Die Behältnisse 1 der Matrix 1M und die den Behältnissen 1 zugeordneten Messeinheiten 10 sind relativ zueinander mechanisch unbeweglich. Bei der hier dargestellten Ausführungsform haben alle Behältnisse 1 einen quadratischen Querschnitt. Gemäß der hier dargestellten Ausführungsform sind auf dem Messträger 22 (Matrix 1M) vierundzwanzig Behältnisse 1 vorgesehen. Andere Anordnungen hinsichtlich Größe und Zahl der Behältnisse 1 in der Matrix 1M sind für einen Fachmann denkbar.

**Figur 5A** zeigt eine Draufsicht auf eine weitere Ausführungsform der Matrix 1M aus mehreren Behältnissen 1. Mit den Messeinheiten 10 des Messträgers 22 können in allen Behältnissen 1 der Matrix 1M die Messungen im Wesentlichen gleichzeitig durchgeführt werden. Diese Messweise kann durchgeführt werden, wenn die Wände 3 der jeweiligen Behältnisse 1 (Wells) für die Wellenlänge der elektromagnetischen Strahlung der Strahlungsquelle 11 und des dadurch erzeugten Streulichts an der Probe 2 nicht transparent ist. In einfachster Weise sind die Wände 3 aus einem nicht transparenten Kunststoff hergestellt.

**Figur 5B** zeigt eine Draufsicht auf die Matrix 1M aus mehreren Behältnissen 1 in starrer Verbindung zueinander, wobei die Behältnisse 1 in einer möglichen Ausführungsform in Gruppen 1₁, 1₂ und 1₃ eingeteilt sind und die Messung in den Gruppen 1₁, 1₂ und 1₃ zu unterschiedlichen Zeiten durchgeführt wird. Die Anzahl der Gruppen 1₁, 1₂ und 1₃ wird nicht als eine Beschränkung der Erfindung angegeben. Sämtliche zu einer Gruppe (1₁, 1₂ oder 1₃) zugehörigen Behältnisse 1 sind durch die gleiche Schraffur gekennzeichnet. Innerhalb der definierten Gruppen 1₁, 1₂ oder 1₃ erfolgt dann die Messung in den Behältnissen 1 simultan, wobei die sukzessive Messung verschiedener Gruppen mit einer Zeitverzögerung kleiner als 2 Sekunden erfolgt. Diese Messweise wird angewendet, wenn in einer Matrixanordnung der Behältnisse 1, deren Wände 3 transparent sind. Dadurch wird eine Weiterleitung von Licht einer Messeinheit 10 auf Sensoren 12 anderer Messeinheiten 10 verhindert. Durch die Anordnung der Gruppen 1₁, 1₂ und 1₃ wird in erster Linie die diagonale Fremdeinkopplung verhindert.

**Figur 5C** zeigt eine Seitenansicht der Matrix 1M, die aus den mehreren, starr verbundenen Behältnissen 1 besteht, auf einer Ausführungsform des Messträgers 22. Der Messträger 22 dient zur Aufnahme der Matrix 1M aus den Behältnissen 1 (Kleinstbioreaktoren). Bei dieser Ausführungsform sind alle Behältnisse 1 während des Messvorgangs abgedeckt. Die Abdeckung 6 ist mit Bohrungen 6B versehen. Dabei ist jedem der Behältnisse 1 eine Bohrung 6B zugeordnet. Die Abdeckung 6 ist eine Sterilbarriere in Form einer Membran oder einer anderen porösen, semipermeablen Schicht. Die Sterilbarriere erlaubt den Gasaustausch in beide Richtungen, wodurch beispielsweise Mikroorganismen mit Sauerstoff versorgt werden oder Stoffwechselprodukte wie CO₂ abgeführt werden. Der Messträger 22 trägt die Vielzahl der Messeinheiten 10 an definierten Positionen, die bei der auf den Messträger 22 eingesetzten Matrix 1M jeweils dem Boden 5 eines jeden Behältnisses 1 ortsfest zugeordnet sind. Der Messträger 22 umfasst ferner ein Elektronikmodul 24, das mit den Messeinheiten 10 in kommunikativer Verbindung steht. Die Energieversorgung der Messeinheiten 10, des Elektronikmoduls 24 und die Datenverbindung 23 erfolgt nach einer im Stand der Technik bekannten Weise.

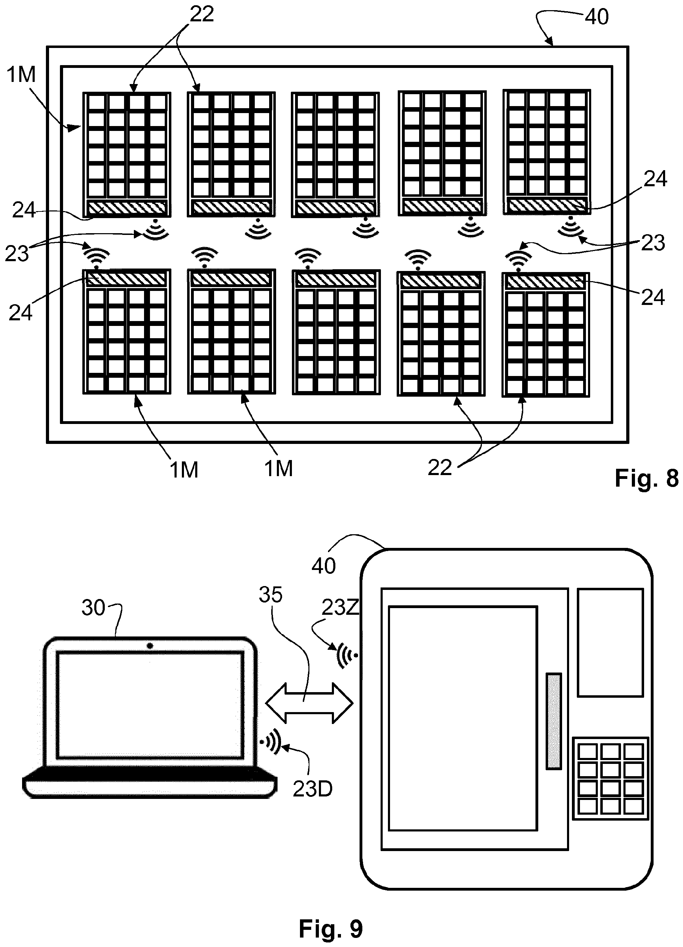

**Figur 6A** zeigt eine Draufsicht auf eine weitere Ausführungsform der Matrix 1M aus mehreren Behältnissen 1, wobei die Matrix 1M auf dem Messträger 22 positioniert ist. Auf dem Messträger 22 ist ferner ein Elektronikmodul 24 ausgebildet, das für die Stromversorgung der Messeinheiten 10 und eine Kommunikation zu Messeinheiten 10 (innerhalb eines Sensornetzwerks) auf dem Messträger 22 über herkömmlich bekannte Verbindungstechnologien sorgt. Eine Datenverbindung 23 zu einer Basisstation 30 bzw. Computer (siehe Figur 9) ist vorgesehen. Die Datenverbindung 23 ist bei der hier beschriebenen Ausführungsform eine drahtlose Kommunikation. Durch die Kommunikation mit der Basisstation 30 bzw. Computer (zur Datenverarbeitung / Datenaufzeichnung) können die aktiven, mit einer Probe 2 gefüllten, Behältnisse 1, sowie der mindestens eine Messträger 22 des Messsystems zu einem kommunizierenden Netzwerk der Messeinheiten 10, zusammengefasst werden.

**Figur 6B** zeigt eine Schnittansicht entlang der in Figur 6A gekennzeichneten Schnittlinie A-A des Messträgers 22 und der aufgesetzten Matrix 1M. Der Messträger 22 ist auf der einer Bewegungseinrichtung 25 platziert, damit den ortsfest mit den Messträger 22 verbundenen Matrix 1M der Behältnisse 1 eine definierte Bewegung aufgeprägt werden kann. Für die Messung an der Probe 2 in den einzelnen Behältnissen 1 und der Bestimmung mindestens einer Veränderlichen der Probe 2, kann eine ununterbrochene, definierte, radiale, und orthogonal zur Schwerkraft verlaufenden Bewegung der Matrix 1M der Behältnisse 1 aus dem Messträger 22 um eine feste Achse A erfolgen. Die Bewegung setzt sich zumindest aus Bewegungskomponenten in X-Koordinatenrichtung X und/oder Y-Koordinatenrichtung Y zusammen. Die Matrix 1M aus den einzelnen Behältnissen 1 sind auf dem Messträger 22 ortsfest den Messeinheiten 10 des Messträgers 22 für die Bestimmung der mindestens einen Veränderlichen der Probe 2 zugeordnet. Mit dem Elektronikmodul 24 bzw. die Datenverbindung 23 werden die Messwerte der Messeinheiten 10 an die Basisstation 30 (siehe Figur 9) übermittelt.

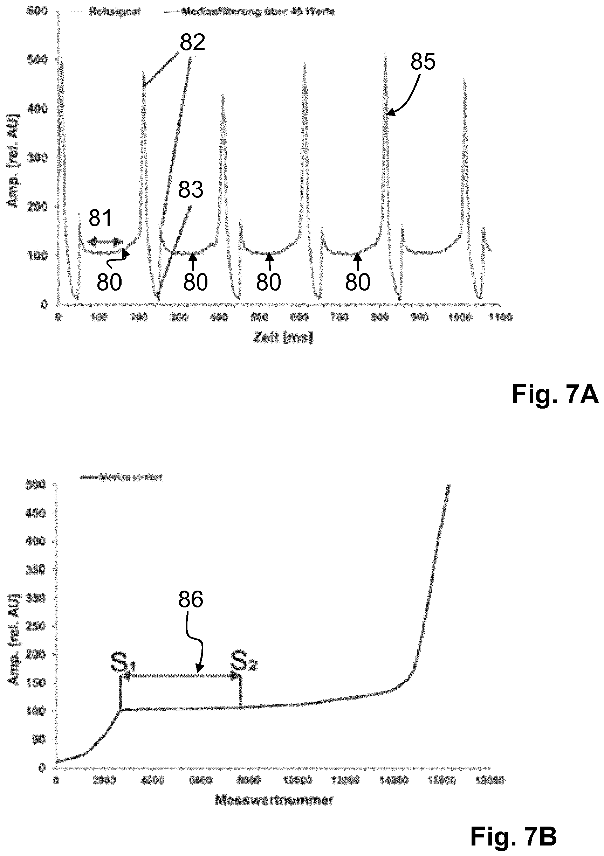

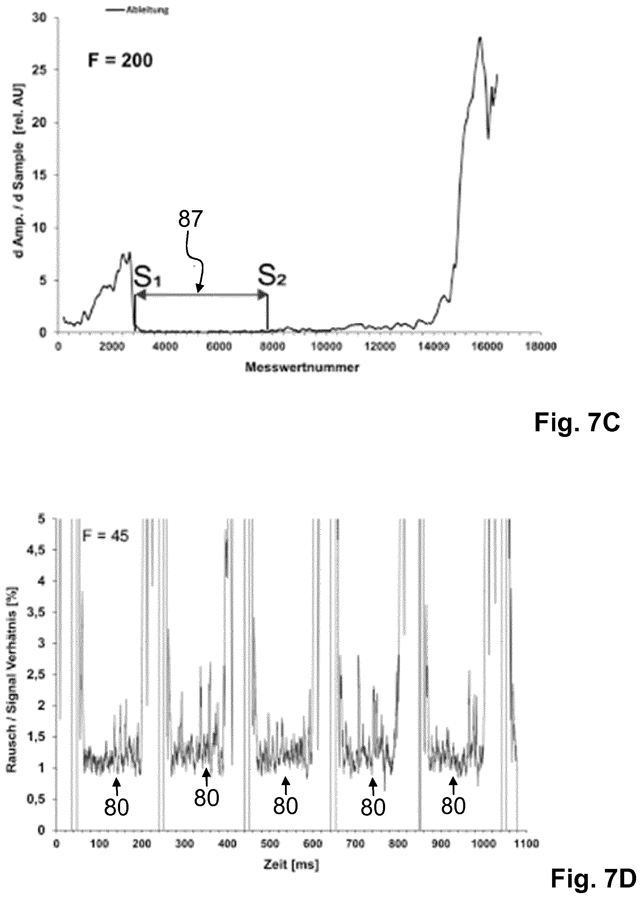

**Figur 7A** zeigt eine graphische Darstellung eines typischen Streulichtsignals einer Zellsuspension konstanter optischer Dichte, das durch periodisch wiederkehrende Blöcke charakterisiert ist. In Folge der Bewegung der Behältnisse 1 (Kleinstbioreaktoren) um eine feste Achse A treten Ungleichverteilungen der flüssigen Probe 2 in den Behältnissen 1 auf (siehe Fig. 1A bis 1C). Die flüssige Probe 2 wird durch Zentripetalkräfte in Richtung der Wände 3 eines jeden Behältnisse der Matrix 1M verlagert. Unter Annahme hoher Bewegungsfrequenzen und geringen Probenvolumina können Fluidverteilungen entstehen, bei denen größere Bereiche des Bodens 5 des Behältnisses 1 nicht von Flüssigkeit bedeckt sind und daher nicht für die Messung geeignet sind. Anhand der kontinuierlichen Messung des Streulichtes, bei einer hohen Messfrequenz von wenigstens 10 kHz, kann über einen definierten Zeitraum das sich periodisch verändernde Flüssigkeitsvolumen der Probe 2 über der Messeinheit 10 zeitlich aufgelöst werden. Das typische Signal einer Zellsuspension konstanter optischer Dichte ist charakterisiert durch periodisch wiederkehrende Blöcke (ähnlich einer Rechteckmodulation) mit Bereichen 80 geringer Signalabweichung über ein definierbares Zeitintervall 81, Signalpeaks 82 im Randbereich der Blöcke sowie Lücken 83 geringer Streulichtamplitude. Von Relevanz sind jene Messbereiche, die während der Bewegung durch den Übergang der flüssigen Probe 2 oberhalb der Messeinheit 10 erzeugt werden. Im Zeitintervall des größten Flüssigkeitsvolumenelements oberhalb der Messeinheit 10 ist die Distanz der Wasser-/Luftgrenzfläche statistisch am größten, wodurch Grenzflächenreflexionen statistisch seltener auf die Messeinheit 10 treffen bzw. durch die optische Wegstrecke innerhalb der Probe 2 gedämpft werden und im geringeren Maße zur Störung des Streulichtsignals beitragen. Weitere Störungen in Form eines kontinuierlichen Messrauschens können während der Bewegung der Probe 2 durch Luftblasen, Schaum und die Inhomogenität der Probe verursacht werden. Zur Filterung des Rohsignals, d. h. Reduktion des Messrauschens kommen Median-Filter oder Savitzky-Golay-Filter einer definierten Fensterbreite zum Einsatz. Nach der Vorfilterung werden relevante Messbereiche anhand der unten angegebenen Kriterien oder einer Kombination aus mehreren Kriterien selektiert und extrahiert. Dadurch erhält man eine geringe Standardabweichung des Streulichtsignals 85 innerhalb eines definierten Zeitintervalls ("Plateaus"; Bereich 80). Ferner ergibt sich ein geringes Signal-Rausch-Verhältnis relativ zur Höhe des Streulichtsignals (siehe Figur 7D) in den Bereichen 80.

**Figur 7B** zeigt eine graphische Darstellung der Sortierung von akkumulierten Messwerten gleicher Streulichtamplituden. In diesem Schritt wird vorhandenes Messrauschen im Rohsignal durch einen der vorstehend angegebenen Filterverfahren reduziert. Dann werden die gefilterten Messdaten der Streulichtamplitude entsprechend von kleinen in Richtung großer Werte sortiert. Durch die Sortierung von akkumulieren Messwerten gleicher Streulichtamplituden bildet sich ein zusammenhängendes Intervall 86 zwischen S1 - S2 aus. S1 und S2 sind Parameter des Verfahrens und können vor und während der physikalischen Erfassung des Streulichtes angegeben werden oder automatisiert durch Ableitung (siehe Figur 7C) des sortierten Streulichtsignalverlaufs ermittelt werden.

**Figur 7C** zeigt eine graphische Darstellung der Ableitung des sortierten Streulichtsignalverlaufs aus Figur 7B. Durch die Ableitung kann ein Threshold (Schwellwert) für die maximale Steigung des sortierten Streulichtsignalverlaufs (Kurvenverlaufs) angegeben werden. Dadurch wird ein zusammenhängendes Intervall 87 geringster Steigung ermittelt. Anschließend wird das arithmetische Mittel aller Messdaten innerhalb des Intervalls gebildet, wobei ein neuer Messwert zur Umrechnung in mindestens eine der Referenzgrößen OD600, Biomassenkonzentration und Zellkonzentration entsteht.

**Figur 7D** zeigt eine graphische Darstellung des Messrauschens. Parallel zur physikalischen Erfassung des Streulichtes wird vorhandenes Messrauschen im Rohsignal durch einen der vorstehend angegebenen Filterverfahren reduziert.

**Figur 8** zeigt die Anordnung von mehreren Messträgern 22 mit jeweils einer darauf angeordneten Matrix 1M aus mehreren Behältnissen 1 in einem Inkubator 40. Bei dem hier dargestellten Messsystem sind im Inkubator 40 zehn Messträger 22 (Messeinheiten) mit einer Matrix 1M aus jeweils vierundzwanzig darauf angeordneten Behältnissen 1 für die Proben 2 eingebracht. Man erreicht dadurch die kontinuierliche, optische Messung und Aufzeichnung von Streulicht, das am biologischen Material in den einzelnen Behältnissen 1 infolge der Bestrahlung mit Licht entsteht. Mittels eines einzigen Messträgers 22 kann eine unterbrechungsfreie, nicht-invasive und simultane Messung an den vierundzwanzig Behältnissen 1 pro Messträger 22 während des Einsatzes in Inkubatoren 40 für Bakterien- und Säugerzellkulturen im radialen Schüttelbetrieb durchgeführt werden. Durch Miniaturisierung der Messträger 22 können bis zu zehn Messträger 22 innerhalb einer Schüttler-/Inkubationsumgebung zeitgleich angeordnet und betrieben werden. Die Kommunikation der einzelnen den Behältnissen 1 des jeweiligen Trägers 22 (Messeinheit) zugeordneten Messeinheiten 10 wird durch das Elektronikmodul 24 gesteuert. Die Kommunikation der Messeinheiten 10 erfolgt über eine jeweils eine Datenverbindung 23, wie z.B. eine Funkverbindung (Bluetooth, WLAN).

**Figur 9** zeigt eine schematische Anordnung eines Inkubators 40 in Verbindung mit einer Basisstation bzw. Computer 30, der für die Aufnahme und Auswertung der Messergebnisse der Substanzen in den Behältnissen 1 (Kleinstbioreaktoren) sorgt. Zur Unterstützung der in einer jeweiligen Untersuchung interessierenden Prozesse können die Messträger 22 mit den mehreren Behältnissen 1 im Inkubator 40 bewegt werden. Dies geschieht bevorzugt mechanisch. Entsprechende, allgemein bekannte Geräte sind beispielsweise Schüttler, Shaker oder Rocker. Solche Geräte sind kommerziell in verschiedenen Ausführungsformen erhältlich, welche ein Behältnis 1, aber auch eine Vielzahl von Behältnissen 1 auf einem Messträger 22 simultan in definierter Weise bewegen können. All diese Geräte finden im Inkubator 40 Platz. Die Basisstation 30 ist über eine bidirektionale Kommunikationsverbindung 35 mit dem Inkubator 40 verbunden, um Daten von den Messträgern 22 im Inkubator 40 zu empfangen und z.B. Steuerungsdaten von der Basisstation 30 an den Inkubator 40 selbst bzw. an die Elektronikmodule 24 der Träger 22 zu senden.

Gemäß einer bevorzugten Ausführungsform verfügt jede Messeinheit 10 über eine Datenverbindung 23, die ein Funktransmitter/-receiver ist, mit dem ein lokales Funknetzwerk zu einer fest stationierten, zentralen Datenverbindung 23Z, ebenfalls ein Funktransmitter/-receiver, hergestellt wird. Bei der verwendeten Datentransfertechnologie kann beispielsweise Bluetooth oder WLAN genutzt werden. Alle Messeinheiten 10 verfügen weiterhin über einen geräteinternen, dauerhaften Datenspeicher zur Aufzeichnung von Messdaten. Der zentrale Funktransmitter/-receiver ist über eine Datenschnittstelle 23D mit einer Basisstation 30 (Gerät zur Datenverarbeitung / Datenaufzeichnung), wie beispielsweise einem Rechner, wie z.B. einem Desktop-Rechner, einem Notebook-Computer, einem Tablet-Computer oder einem Smart-Phone, verbunden.

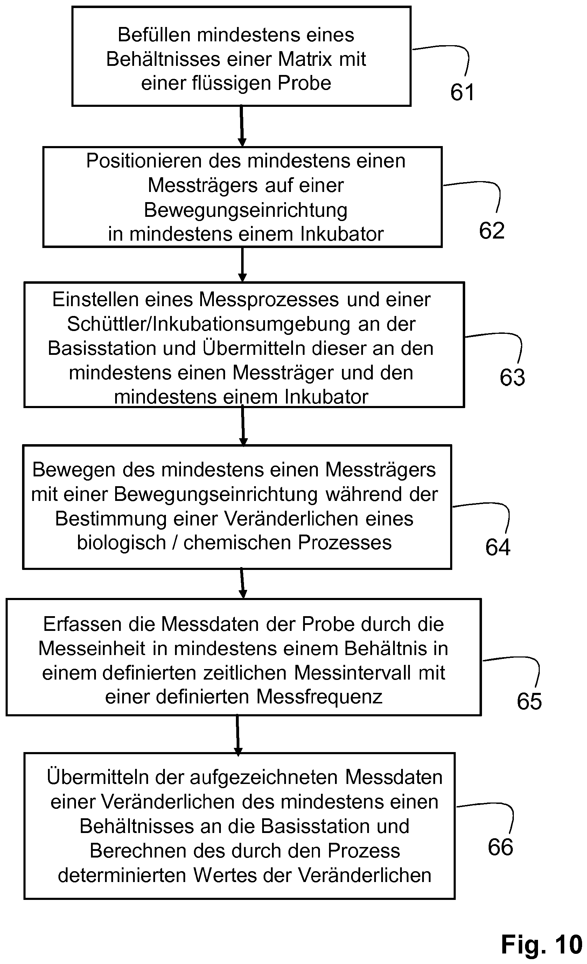

Ein Ablaufdiagramm des erfindungsgemäßen Verfahrens der parallelisierten Erfassung von Zell- und Biomassekonzentrationen von Zellkulturen (flüssige Probe 2) ist in **Figur 10** dargestellt. In einem Schritt 61 zu Beginn des erfindungsgemäßen Verfahrens erfolgt das Befüllen mindestens eines Behältnisses 1 eines Trägers 22 mit einer flüssigen Probe 2 (Beschaffung und Eigenschaft der Probe ist hinreichend oben beschrieben). Die Behältnisse 1 sind dabei regelmäßig in Spalten 9 und Zeilen 8 (in Form einer Matrix) ortsfest auf dem Träger angeordnet. Die Öffnung 4 der Behältnisse 1 kann mit einer Abdeckung 6 verschlossen werden, damit beim Messprozess die flüssige Probe 2 nicht über das Behältnis 1 hinaustritt.

In einen nächsten Schritt 62 wird der mindestens eine Messträger 22 auf einer Bewegungseinrichtung 25 in mindestens einem Inkubator 40, der mit der Basisstation 30 kommunikativ verbunden ist, eingebracht. Es ist angemerkt, dass in einer anderen Ausführungsform des Verfahrens auch auf den Inkubator verzichtet werden kann.

Im Schritt 63 erfolgt das Einstellen eines Messprozesses und einer Schüttler-/Inkubationsumgebung an der Basisstation 30. Die Einstellungen werden an den mindestens einen Messträger 22 und ggf. an den mindestens einen Inkubator 40 (falls notwendig auch an die Bewegungseinrichtung 25) übermittelt. Das Einstellen des Messprozesses kann z.B. ohne darauf beschränkt zu sein, die Inkubationsbedingungen, das radiale Bewegungsmuster (wie z.B. Wiederholungs-Frequenz und Drehsinn) da Bewegungsart "radial" vorher vorgegeben wird) der Bewegungseinrichtung 25, die Steuerung der Strahlungsquelle 11, die Definition der Messfrequenz für ein zeitliches Messintervall (zur Erzeugung eines Messwerts stellt der Anwender nur ein, dass z.B. alle 10 Sekunden ein Messwert erfasst werden soll) oder die Einstellung der von der Strahlungsquelle 11 ausgesendeten Wellenlänge umfassen.

Im Schritt 64 wird das Bewegen des mindestens einen Trägers 22 mit der diesem zugeordneten Bewegungseinrichtung 25 durchgeführt. Während der Bewegung des Trägers 22 nach einem definierten Bewegungsmuster, erfolgt die Bestimmung einer Veränderlichen des biologischen, chemischen oder biochemischen Prozesses. Die Bewegung des Trägers 22 wird erfindungsgemäß ununterbrochen und mit einer definierten, radialen und orthogonal zur Schwerkraft S verlaufenden Bewegung um eine feste Achse A durchgeführt.

In einem zu Schritt 64 zeitlich parallelen Schritt 65 erfolgt durch die Messeinheit 10 das Erfassen der Messdaten der von in dem mindestens einen Behältnis 1 vorhandenen flüssigen und bewegten Probe 2. Das Erfassen der Messdaten erfolgt in einem definierten zeitlichen Messintervall mit einer definierten Messfrequenz von mindestens 10 kHz. In jedem Behältnis 1, in dem sich eine Probe befindet, werden die Messdaten mit dem Sensor der Messeinheit 10 aufgenommen. Den jeweiligen Behältnissen 1 ist jeweils eine Messeinheit 10 fest zugeordnet, wobei die Messeinheiten 10 ortsfest auf dem Messträger 22 für die Behältnisse 1 (z.B. Mikrotiterplatte) angeordnet sind. Die Messeinheit 10 besteht aus der steuerbaren Strahlungsquelle 11 und dem mindestens einem Sensor 12.

Im Schritt 66 erfolgt letztendlich das Übermitteln der aufgezeichneten Messdaten einer Veränderlichen in dem mindestens einen Behältnis 1. Die Messdaten werden dabei an die Basisstation 30 (oder einer geeigneten Auswerteeinheit) aus dem Inkubator 40 heraus übermittelt. Mit der Basisstation 30 erfolgt das Berechnen des durch den Auswerteprozess determinierten Wertes der Veränderlichen. Bei der Veränderlichen handelt es sich z. B. um die Trübung und die optische Dichte flüssiger Proben, sowie im Speziellen die Zelldichte, Biomasse- und Zellkonzentration, pH-Wert, O₂-Sättigung der Flüssigkeit und die Umgebungstemperatur. Für die Bestimmung des pH-Werts oder der O₂-Sättigung der Flüssigkeit werden Sensorpads (hier nicht dargestellt) in das Behältnis eingeklebt. Der pH-Wert oder die O₂-Sättigung werden als optische Antwort von dem jeweiligen Behältnis zugeordneten Sensor 12 erfasst, der zuvor mit einer Lichtquelle beleuchtet wurde. Die relative Sättigung an gelöstem Sauerstoff in der jeweiligen Probe 2 wird durch eine Veränderung des Energieeintrags bei der Bewegung der Behältnisse 1 bzw. des Trägers 22 durch das Bewegungsmuster der Bewegungseinrichtung 25 geregelt. Besonders vorteilhaft ist, wenn die aufgezeichneten Messdaten mit einer Datenverbindung 23 (z.B. über Funk) vom Inkubator 40 zu der Basisstation 30 übertragen werden, da die Fehlerquelle eines Kabelbruchs wegfällt.

### Bezugszeichenliste:

- 1: Behältnis
- 1₁, 1₂, 1₃: Gruppe
- 1M: Matrix
- 2: Probe
- 3: Wand
- 3T: Teilabschnitt
- 4: Öffnung
- 5: Boden
- 5F: Bodenfläche
- 6: Abdeckung
- 6B: Bohrung
- 7: Ecke
- 8: Zeile
- 9: Spalte
- 10: Messeinheit
- 11: Strahlungsquelle
- 11S: Strahl
- 12: Sensor
- 13: optisches System
- 14: Winkel
- 15: optische Faser
- 16: optischer Filter
- 17: Messbereich
- 18: Querschnitt
- 19: Winkel
- 20: Licht
- 22: Messträger
- 23: Datenverbindung
- 23D: Datenschnittstelle
- 23Z: zentrale Datenverbindung
- 24: Elektronikmodul
- 25: Bewegungseinrichtung
- 26: Leuchtdiode
- 27: Lichtzylinder
- 29: Winkel
- 30: Basisstation
- 35: bidirektionale Kommunikationsverbindung
- 40: Inkubator
- 50: Signalmuster
- 51: zeitliche Ableitung
- 61: Schritt
- 62: Schritt
- 63: Schritt
- 64: Schritt
- 65: Schritt
- 66: Schritt
- 71: Abstandshalter
- 72: Lochblende
- 73: Abstandshalter
- 74: optische Linse
- 75: Abstandshalter
- 80: Bereich
- 81: Zeitintervall
- 82: Signalpeak
- 83: Lücke
- 85: Streulichtsignal
- 86: Intervall
- 87: Intervall
- A: Achse
- A-A: Schnittlinie
- O: Orthogonale
- oA: optische Achse
- R: orthogonale Richtung
- S: Schwerkraft
- S₁: Parameter
- S₂: Parameter
- X: X-Koordinatenrichtung
- Y: Y-Koordinatenrichtung
- Z: Z-Koordinatenrichtung

## Patentansprüche

1. Vorrichtung zur Erfassung mindestens einer Veränderlichen von flüssigen Proben (2) während eines biologischen, chemischen oder biochemischen Prozesses, umfassend:
einen Messträger (22), der in X-Koordinatenrichtung (X) und in Y-Koordinatenrichtung (Y) bewegbar ist, in dem mehrere Messeinheiten (10) angeordnet sind, wobei jede Messeinheit (10) mindestens eine steuerbare Strahlungsquelle (11) elektromagnetischer Strahlung und mindestens einen Sensor (12) zur Detektion elektromagnetischer Strahlung umfasst; und
eine Matrix (1M) aus mehreren Behältnissen (1) mit jeweils quadratischer Querschnittsform, wobei jedes der Behältnisse (1) aus einem Boden (5) und vier Wänden (3) besteht, wobei zwei zusammentreffende Wände jeweils eine Ecke (7) definieren;
**dadurch gekennzeichnet,**
**dass** die Matrix (1M) der Behältnisse (1) derart mit dem Messträger (22) lösbar verbunden ist, dass bei der mit dem Messträger (22) verbundenen Matrix (1M) jedem Behältnis (1) der Matrix (1M) je eine Messeinheit (10) im Bereich einer Ecke (7) am Boden (5) des Behältnisses (1) zugeordnet ist, wobei die Bewegung des Messträgers (22) in X-Koordinatenrichtung (X) und in Y-Koordinatenrichtung (Y) einen Messbereich (17) bildet, der eine Anhäufung der flüssigen Probe (2) in derjenigen Ecke (7) ist, die der Messeinheit (10) zugeordnet ist;
**dass** die Strahlungsquelle (11) aus mindestens einer Leuchtdiode (26) und einem der mindestens einen Leuchtdiode (26) nachgeordneten optischen System (13) zur Lenkung und Formung der elektromagnetischen Strahlung besteht, wobei durch das optische System ein Lichtzylinder (27) im Bereich der Ecke (7), an der die Anhäufung der flüssigen Probe (2) ist, ausbildbar ist; und
**dass** der Sensor (12) dafür ausgebildet ist, Streulicht von der flüssigen Probe (2) aus dem Bereich der Ecke (7) zu empfangen.

2. Vorrichtung nach Anspruch 1, wobei der Boden (5) eines jeden Behältnisses (1) der Matrix (1M) derart ausgebildet ist, dass er für die elektromagnetische Strahlung von der steuerbaren Strahlungsquelle (11) in die flüssige Probe (2) und für das Steulicht aus der flüssigen Probe zu dem mindestens einen Sensor (12) durchlässig ist.

3. Vorrichtung nach Anspruch 1, wobei das optische System (13) zur Lenkung und Formung der elektromagnetischen Strahlung zumindest eine Lochblende (72) und eine optische Linse (74) umfasst, wobei die Linse (74) die elektromagnetische Strahlung in der flüssigen Probe zu einem Zylinder (27) kollimiert.

4. Vorrichtung nach einem der vorangehenden Ansprüche, wobei der mindestens eine Sensor (12) mit einer optischen Faser (15) und mit mindestens einem optischen Filter (16) versehen ist und wobei die Strahlungsquelle (11) und die optische Faser (15) des Sensors (12) in der Messeinheit (10) unter einem Winkel (29) zueinander angeordnet sind.

5. Vorrichtung nach Anspruch 1, wobei der Messträger (22) mit einem Elektronikmodul (24) versehen ist, das mit dem mindestens einen Sensor (12) jeder Messeinheit (10) kommunikativ verbunden ist, und wobei das Elektronikmodul (24) über eine Datenverbindung (23) mit einer Basisstation (30) verbindbar ist.

6. Verfahren zur Erfassung mindestens einer Veränderlichen einer flüssigen Probe (2) während eines biologischen, chemischen oder biochemischen Prozesses, wobei eine Matrix (1M) aus mehreren Behältnissen (1) mit jeweils quadratischer Querschnittsform vorgesehen ist, und jedes der Behältnisse (1) der Matrix (1M) aus einem Boden (5) und vier Wänden (3) besteht, wobei zwei zusammentreffende Wände jeweils eine Ecke (7) definieren, und das Verfahren die folgenden Schritte umfasst:
• Befüllen von mindestens einem Behältnis (1) der Matrix (1M) mit der flüssigen Probe (2);
• Aufsetzen der Matrix (1M) auf einen Messträger (22) mit mehreren Messeinheiten (10), wobei jeweils eine der mehreren Messeinheiten (10) im Messträger (22), die mindestens eine steuerbare Strahlungsquelle (11) elektromagnetischer Strahlung und mindestens einen Sensor (12) zur Detektion elektromagnetischer Strahlung umfasst, einer Ecke (7) am Boden (5) eines jeden Behältnisses (1) der Matrix (1M) ortsfest zugeordnet ist;
• Bewegen des Messträgers (22) in X-Koordinatenrichtung (X) und in Y-Koordinatenrichtung (Y) und dabei Bestimmen der mindestens einen Veränderlichen während des biologischen, chemischen oder biochemischen Prozesses in dem mindestens einem Behältnis (1) der Matrix (1M);
o wobei die Bewegung des Messträgers (22) ununterbrochen und mit einer definierten, radialen und orthogonal zur Schwerkraft (S) verlaufenden Bewegung um eine feste Achse (A) durchgeführt wird,
o wobei in jedem Behältnis (1) der Matrix (1M) im Bereich der Ecke (7) aufgrund der Bewegung ein Messbereich (17) ausgebildet wird und pro befülltem Behältnis (1) mittels der mindestens einen steuerbaren Strahlungsquelle (11) elektromagnetische Strahlung in Form eines Lichtzylinders (27) durch den Boden (5) in den Messbereich (17) eingestrahlt wird; und wobei mindestens einer der Sensoren (12) durch den Boden (5) Streulicht aus der flüssigen Probe (2) aus dem Messbereich (17) im Bereich der Ecke (7) erfasst.

7. Verfahren nach Anspruch 6, wobei ein von der Strahlungsquelle (11) ausgehender Strahl (11S) unter einem Winkel (14) durch den Boden (5) in das jeweilige Behältnis (1) der Matrix (1M) eingestrahlt wird und wobei eine optische Faser (15) des optischen Sensors (12) unter einem Winkel (19) zu einer Orthogonalen (O) des Bodens (5) angeordnet ist und elektromagnetische Strahlung aus der flüssigen Probe (2) durch den Boden (5) hindurch empfängt.

8. Verfahren nach einem der Ansprüche 6 bis 7, wobei die Behältnisse (1) der Matrix (1M) derart mit den ihnen zugeordneten Messeinheiten (10) des Messträgers (22) vermessen werden, dass die Behältnisse (1) gruppiert und Messwerte aus den gruppierten Behältnissen (1) zeitversetzt gewonnen werden.

9. Verfahren nach einem der Ansprüche 6 bis 8, wobei die mindestens eine Veränderliche in jedem Behältnis in einem definierten zeitlichen Messintervall mit einer Messfrequenz von mindestens 10000 Messereignissen pro Sekunde aufgezeichnet wird und wobei die aufgezeichneten Messdaten der mindestens einen Veränderlichen eines jeden Behältnisses (1) der Matrix (1M) unabhängig voneinander nach einem mathematischen Verfahren in einem definierten zeitlichen Messintervall verarbeitet und in einen, zeitlich nach Beginn des Prozesses determinierten Wert, der Veränderlichen umgerechnet werden.

10. Verfahren nach Anspruch 8, wobei die zeitversetzt gewonnenen Messwerte mittels einer Datenverbindung (23) zu einer Basisstation (30) übertragen werden.

11. System zur Erfassung mindestens einer Veränderlichen von flüssige Proben (2) während eines biologischen, chemischen oder biochemischen Prozesses, umfassend:
eine Matrix (1M) aus mehreren starr miteinander verbundenen Behältnissen (1), wobei jedes der Behältnisse (1) aus einem Boden (5) und vier Wänden (3) besteht, wobei zwei zusammentreffende Wände jeweils eine Ecke (7) definieren und jedes Behältnis (1) eine quadratische Querschnittsform besitzt;
mehrere Messeinheiten (10), die in einem Messträger (22) angeordnet sind, so dass bei der auf dem Messträger (22) ortsfest positionierten Matrix (1M) jeweils einer Ecke (7) am Boden (5) jedes Behältnisses (1) der Matrix (1M) jeweils eine Messeinheit (10) zugeordnet ist, wobei die Messeinheit (10) eine steuerbare Strahlungsquelle (11) für elektromagnetische Strahlung zu deren Einstrahlung durch den Boden (5) und mindestens einen Sensor (12) zur Detektion der durch den Boden austretenden gestreuten elektromagnetischen Strahlung umfasst;
ein Elektronikmodul (24), das mit jeder Messeinheit (10) des Messträgers (22) zur Steuerung der Strahlungsquelle (11) und zur Detektion der gestreuten elektromagnetischer Strahlung mit dem mindestens einen Sensor (12) der Messeinheit (10) kommunikativ verbunden ist;
eine Bewegungseinrichtung (25), mittels derer der Messträger (22) in X-Koordinatenrichtung (X) und in Y-Koordinatenrichtung (Y) mit einer definierten, radialen und orthogonal zur Schwerkraft (S) verlaufenden Bewegung um eine feste Achse (A) bewegbar ist, wobei
die Bewegung des Messträgers (22) einen Messbereich (17) bildet, der eine Anhäufung der flüssigen Probe (2) in derjenigen Ecke (7) ist, die der Messeinheit (10) zugeordnet ist; und
eine Basisstation (30), die mit dem Elektronikmodul (24) des Messträgers (22) kommunikativ verbunden ist.

12. System nach Anspruch 11, wobei mindestens ein Inkubator (40) vorgesehen ist, in dem die Bewegungseinrichtung (25) und der Messträger (22) aufgenommen sind.

13. System nach einem der Ansprüche 11 - 12, wobei bis zu zehn Messträger (22) auf der Bewegungseinrichtung (25) untergebracht sind, wodurch eine unterbrechungsfreie, nicht-invasive und simultane Messung an mehreren Behältnissen (1) einer Matrix (1M) auf mehreren Messträgern (22) durchführbar ist.

14. System nach einem der Ansprüche 11 - 13, wobei das System mehrere Inkubatoren (40) umfasst und mehrere Messträger (22) in mehreren Inkubatoren positioniert sind, so dass die Messträger (22) unterschiedlichen Inkubationsumgebungen und Bewegungen der Bewegungseinrichtung (25) unterliegen.

## Claims

1. Device for detecting at least one variable of liquid samples (2) during a biological, chemical or biochemical process, comprising:
a measuring carrier (22) movable in an X coordinate direction (X) and in a Y coordinate direction (Y), in which a plurality of measuring units (10) are arranged, wherein each measuring unit (10) comprises at least one controllable radiation source (11) of electromagnetic radiation and at least one sensor (12) for detecting electromagnetic radiation; and
a matrix (1M) of a plurality of containers (1) each having a square cross-sectional shape, wherein each of the containers (1) consists of a bottom (5) and four walls (3), wherein two coinciding walls each define a corner (7);
**characterized in that**
the matrix (1M) of the containers (1) is detachably connected to the measuring carrier (22) in such a way that, with the matrix (1M) connected to the measuring carrier (22), each container (1) of the matrix (1M) is assigned a measuring unit (10) each in the region of a corner (7) on the bottom (5) of the container (1), wherein the movement of the measuring carrier (22) in the X coordinate direction (X) and in the Y coordinate direction (Y) forms a measuring area (17) which is an accumulation of the liquid sample (2) **in that** corner (7) which is assigned to the measuring unit (10);
the radiation source (11) consists of at least one light-emitting diode (26) and an optical system (13), arranged downstream of the at least one light-emitting diode (26), for directing and shaping the electromagnetic radiation, wherein the optical system can form a light cylinder (27) in the region of the corner (7) at which the accumulation of the liquid sample (2) is located; and
the sensor (12) is adapted to receive scattered light from the liquid sample (2) from the region of the corner (7).

2. Device according to claim 1, wherein the bottom (5) of each container (1) of the matrix (1M) is formed such that it is transmissive for the electromagnetic radiation from the controllable radiation source (11) into the liquid sample (2) and for the scattered light from the liquid sample to the at least one sensor (12).

3. Device according to claim 1, wherein the optical system (13) for directing and shaping the electromagnetic radiation comprises at least one pinhole (72) and an optical lens (74), wherein the lens (74) collimates the electromagnetic radiation in the liquid sample into a cylinder (27).

4. Device according to one of the preceding claims, wherein the at least one sensor (12) is provided with an optical fiber (15) and with at least one optical filter (16), and wherein the radiation source (11) and the optical fiber (15) of the sensors (12) are arranged in the measuring unit (10) at an angle (29) to each other.

5. Device according to claim 1, wherein the measuring carrier (22) is provided with an electronic module (24) which is communicatively connected to the at least one sensor (12) of each measuring unit (10), and wherein the electronic module (24) can be connected to a base station (30) via a data link (23).

6. Method for detecting at least one variable of a liquid sample (2) during a biological, chemical or biochemical process, wherein a matrix (1M) of a plurality of containers (1) each having a square cross-sectional shape is provided, and each of the containers (1) of the matrix (1M) consists of a bottom (5) and four walls (3), wherein two coinciding walls each define a corner (7), and the method comprises the following steps:
• filling at least one container (1) of the matrix (1M) with the liquid sample (2);
• placing the matrix (1M) on a measuring carrier (22) having a plurality of measuring units (10), wherein in each case one of the plurality of measuring units (10) in the measuring carrier (22), which comprises at least one controllable radiation source (11) of electromagnetic radiation and at least one sensor (12) for detecting electromagnetic radiation, is assigned in a stationary manner to a corner (7) at the bottom (5) of each container (1) of the matrix (1M);
• moving the measuring carrier (22) in X coordinate direction (X) and in Y coordinate direction (Y) and thus determining the at least one variable during the biological, chemical or biochemical process in the at least one container (1) of the matrix (1M);
∘ wherein the movement of the measuring carrier (22) is performed continuously and with a defined radial movement about a fixed axis (A) orthogonal to the force of gravity (S),
∘ wherein a measuring area (17) is formed in each container (1) of the matrix (1M) in the region of the corner (7) as a result of the movement and electromagnetic radiation in the form of a light cylinder (27) is irradiated through the bottom (5) into the measuring area (17) for each filled container (1) by means of the at least one controllable radiation source (11), and
∘ wherein at least one of the sensors (12) detects scattered light from the liquid sample (2) through the bottom (5) from the measuring area (17) in the region of the corner (7).

7. Method according to claim 6, wherein a beam (11S) emitted from the radiation source (11) is irradiated at an angle (14) through the bottom (5) into the respective container (1) of the matrix (1M), and wherein an optical fiber (15) of the optical sensor (12) is arranged at an angle (19) to an orthogonal (0) of the bottom (5) and receives electromagnetic radiation from the liquid sample (2) through the bottom (5).

8. Method according to one of claims 6 to 7, wherein the containers (1) of the matrix (1M) are measured with the measuring units (10) of the measuring carrier (22) assigned to them in such a way that the containers (1) are grouped and measured values are obtained from the grouped containers (1) with a time delay.

9. Method according to one of claims 6 to 8, wherein the at least one variable in each container is recorded in a defined temporal measurement interval with a measurement frequency of at least 10000 measurement events per second and wherein the recorded measurement data of the at least one variable of each container (1) of the matrix (1M) are processed independently of one another according to a mathematical method in a defined temporal measurement interval and are converted into a value, determined temporally after the start of the process, of the variable.

10. Method according to claim 8, wherein the measured values obtained with a time delay are transmitted to a base station (30) by means of a data link (23).

11. System for detecting at least one variable of liquid samples (2) during a biological, chemical or biochemical process, comprising:
a matrix (1M) consisting of a plurality of rigidly interconnected containers (1), wherein each of said containers (1) consists of a bottom (5) and four walls (3), wherein two coinciding walls each define a corner (7) and each container (1) has a square cross-sectional shape;
a plurality of measuring units (10) which are arranged in a measuring carrier (22) such that, with the matrix (1M) positioned in a stationary manner on the measuring carrier (22), in each case one measuring unit (10) is assigned to a corner (7) at the bottom (5) of each container (1) of the matrix (1M), wherein the measuring unit (10) comprises a controllable radiation source (11) for electromagnetic radiation for the irradiation thereof through the bottom (5) and at least one sensor (12) for the detection of the scattered electromagnetic radiation emerging through the bottom;
an electronic module (24) communicatively connected to each measuring unit (10) of the measuring carrier (22) for controlling the radiation source (11) and for detecting the scattered electromagnetic radiation with the at least one sensor (12) of the measuring unit (10);
a movement device (25), by means of which the measuring carrier (22) is movable in X coordinate direction (X) and in Y coordinate direction (Y) with a defined radial movement orthogonal to the force of gravity (S) about a fixed axis (A), wherein the movement of the measuring carrier (22) forms a measuring area (17) which is an accumulation of the liquid sample (2) in that corner (7) which is assigned to the measuring unit (10); and
a base station (30) communicatively connected to the electronic module (24) of the measuring unit (22).

12. System according to claim 11, wherein at least one incubator (40) is provided in which the movement device (25) and the measuring carrier (22) are accommodated.

13. System according to any one of claims 11 to 12, wherein up to ten measuring carriers (22) are accommodated on the movement device (25), whereby an uninterrupted, non-invasive and simultaneous measurement on a plurality of containers (1) of a matrix (1M) can be performed on a plurality of measuring carriers (22).

14. System according to any one of claims 11 to 13, wherein the system comprises a plurality of incubators (40) and a plurality of measuring carriers (22) are positioned in a plurality of incubators such that the measuring carriers (22) are subject to different incubation environments and movements of the movement device (25).

## Revendications

1. Dispositif pour détecter au moins une variable d'échantillons liquides (2) pendant un processus biologique, chimique ou biochimique, comprenant :
un support de mesure (22) qui est mobile dans une direction des coordonnées X (X) et dans une direction des coordonnées Y (Y), dans lequel sont disposées plusieurs unités de mesure (10), chaque unité de mesure (10) comprenant au moins une source de rayonnement commandable (11) de rayonnement électromagnétique et au moins un capteur (12) pour la détection de rayonnement électromagnétique ; et
une matrice (1M) constituée de plusieurs récipients (1) ayant chacun une forme carrée en coupe transversale, chacun des récipients (1) étant constitué d'un fond (5) et de quatre parois (3), deux parois qui se rejoignent définissant chaque fois un coin (7) ;
**caractérisé en ce que**
la matrice (1M) des récipients (1) est reliée au support de mesure (22) de manière amovible de telle sorte que, lorsque la matrice (1M) est reliée au support de mesure (22), une unité de mesure (10) est associée à chaque récipient (1) de la matrice (1M) dans la zone d'un coin (7) sur le fond (5) du récipient (1), le mouvement du support de mesure (22) dans la direction des coordonnées X (X) et dans la direction des coordonnées Y (Y) formant une zone de mesure (17) qui est une accumulation de l'échantillon liquide (2) dans le coin (7) qui est associé à l'unité de mesure (10) ;
la source de rayonnement (11) est constituée d'au moins une diode électroluminescente (26) et d'un système optique (13) disposé en aval de ladite au moins une diode électroluminescente (26) pour diriger et mettre en forme le rayonnement électromagnétique, un cylindre lumineux (27) pouvant être formé par le système optique dans la zone du coin (7) où se trouve l'accumulation de l'échantillon liquide (2) ; et
le capteur (12) est conçu pour recevoir de la lumière diffusée par l'échantillon liquide (2) provenant de la zone du coin (7).

2. Dispositif selon la revendication 1, dans lequel le fond (5) de chaque récipient (1) de la matrice (1M) est conçu de telle sorte qu'il est transparent au rayonnement électromagnétique provenant de la source de rayonnement commandable (11) dans l'échantillon liquide (2) et à la lumière diffusée provenant de l'échantillon liquide vers ledit au moins un capteur (12).

3. Dispositif selon la revendication 1, dans lequel le système optique (13) pour diriger et mettre en forme le rayonnement électromagnétique comprend au moins un diaphragme à trou (72) et une lentille optique (74), la lentille (74) collimatant le rayonnement électromagnétique dans l'échantillon liquide en un cylindre (27).

4. Dispositif selon l'une des revendications précédentes, dans lequel ledit au moins un capteur (12) est pourvu d'une fibre optique (15) et d'au moins un filtre optique (16), et dans lequel la source de rayonnement (11) et la fibre optique (15) des capteurs (12) sont disposées dans l'unité de mesure (10) selon un angle (29) l'une par rapport à l'autre.

5. Dispositif selon la revendication 1, dans lequel le support de mesure (22) est pourvu d'un module électronique (24) qui est relié de manière communicante audit au moins un capteur (12) de chaque unité de mesure (10), et dans lequel le module électronique (24) peut être relié à une station de base (30) par une liaison de données (23).

6. Procédé pour détecter au moins une variable d'un échantillon liquide (2) pendant un processus biologique, chimique ou biochimique, dans lequel est prévue une matrice (1M) constituée de plusieurs récipients (1) ayant chacun une forme carrée en coupe transversale, et chacun des récipients (1) de la matrice (1M) est constitué d'un fond (5) et de quatre parois (3), deux parois qui se rejoignent définissant chaque fois un coin (7), le procédé comprenant les étapes consistant à :
• remplir au moins un récipient (1) de la matrice (1M) avec l'échantillon liquide (2) ;
• mettre en place la matrice (1M) sur un support de mesure (22) avec plusieurs unités de mesure (10), l'une respective des plusieurs unités de mesure (10) dans le support de mesure (22), qui comprend au moins une source de rayonnement commandable (11) de rayonnement électromagnétique et au moins un capteur (12) pour la détection de rayonnement électromagnétique, étant associée de manière fixe à un coin (7) sur le fond (5) de chaque récipient (1) de la matrice (1M) ;
• déplacer le support de mesure (22) dans la direction des coordonnées X (X) et dans la direction des coordonnées Y (Y) et déterminer ainsi ladite au moins une variable pendant le processus biologique, chimique ou biochimique dans ledit au moins un récipient (1) de la matrice (1M) ;
o le mouvement du support de mesure (22) étant effectué de manière ininterrompue et avec un mouvement défini, radial et orthogonal à la force de gravité (S) autour d'un axe fixe (A),
o une zone de mesure (17) étant formée dans chaque récipient (1) de la matrice (1M) dans la zone du coin (7) en raison du mouvement et un rayonnement électromagnétique sous la forme d'un cylindre lumineux (27) étant émis à travers le fond (5) dans la zone de mesure (17) par récipient (1) rempli au moyen de ladite au moins une source de rayonnement commandable(11) ; et
o au moins l'un des capteurs (12) détectant, à travers le fond (5), de la lumière diffusée par l'échantillon liquide (2) provenant de la zone de mesure (17) dans la zone du coin (7).

7. Procédé selon la revendication 6, dans lequel un faisceau (11S) issu de la source de rayonnement (11) est émis selon un angle (14) à travers le fond (5) dans le récipient (1) respectif de la matrice (1M), et dans lequel une fibre optique (15) du capteur optique (12) est disposée selon un angle (19) par rapport à une orthogonale (O) du fond (5) et reçoit le rayonnement électromagnétique provenant de l'échantillon liquide (2) à travers le fond (5).

8. Procédé selon l'une des revendications 6 à 7, dans lequel les récipients (1) de la matrice (1M) sont mesurés avec les unités de mesure (10) du support de mesure (22) qui leur sont associées de telle sorte que les récipients (1) sont regroupés et que des valeurs de mesure sont obtenues avec un décalage temporel à partir des récipients (1) regroupés.

9. Procédé selon l'une des revendications 6 à 8, dans lequel ladite au moins une variable dans chaque récipient est enregistrée dans un intervalle de mesure temporel défini avec une fréquence de mesure d'au moins 10 000 événements de mesure par seconde et dans lequel les données de mesure enregistrées de ladite au moins une variable de chaque récipient (1) de la matrice (1M) sont traitées indépendamment les unes des autres selon un procédé mathématique dans un intervalle de mesure temporel défini et sont converties en une valeur de la variable déterminée dans le temps après le début du processus.

10. Procédé selon la revendication 8, dans lequel les valeurs de mesure obtenues avec un décalage temporel sont transmises à une station de base (30) au moyen d'une liaison de données (23).

11. Système pour détecter au moins une variable d'échantillons liquides (2) pendant un processus biologique, chimique ou biochimique, comprenant :
une matrice (1M) de plusieurs récipients (1) reliés rigidement les uns aux autres, chacun des récipients (1) étant constitué d'un fond (5) et de quatre parois (3), deux parois qui se rejoignent définissant chaque fois un coin (7) et chaque récipient (1) ayant une forme carrée en coupe transversale ;
plusieurs unités de mesure (10) qui sont disposées dans un support de mesure (22), de telle sorte que, pour la matrice (1M) positionnée de manière fixe sur le support de mesure (22), une unité de mesure (10) est chaque fois associée à un coin (7) sur le fond (5) de chaque récipient (1) de la matrice (1M), l'unité de mesure (10) comprenant une source de rayonnement commandable (11) de rayonnement électromagnétique pour son émission à travers le fond (5) et au moins un capteur (12) pour la détection du rayonnement électromagnétique diffusé sortant à travers le fond ;
un module électronique (24) relié de manière communicante à chaque unité de mesure (10) du support de mesure (22) pour commander la source de rayonnement (11) et pour détecter le rayonnement électromagnétique diffusé avec ledit au moins un capteur (12) de l'unité de mesure (10) ;
un dispositif de déplacement (25) au moyen duquel le support de mesure (22) peut être déplacé dans la direction des coordonnées X (X) et dans la direction des coordonnées Y (Y) avec un mouvement défini, radial et orthogonal à la force de gravité (S) autour d'un axe fixe (A), le mouvement du support de mesure (22) formant une zone de mesure (17) qui est une accumulation de l'échantillon liquide (2) dans le coin (7) qui est associé à l'unité de mesure (10) ; et
une station de base (30) reliée de manière communicante au module électronique (24) du support de mesure (22).

12. Système selon la revendication 11, dans lequel il est prévu au moins un incubateur (40) dans lequel sont reçus le dispositif de déplacement (25) et le support de mesure (22).

13. Système selon l'une des revendications 11 à 12, dans lequel jusqu'à dix supports de mesure (22) sont logés sur le dispositif de déplacement (25), ce qui permet de réaliser une mesure sans interruption, non invasive et simultanée sur plusieurs récipients (1) d'une matrice (1M) sur plusieurs supports de mesure (22).

14. Système selon l'une des revendications 11 à 13, dans lequel le système comprend plusieurs incubateurs (40) et plusieurs supports de mesure (22) sont positionnés dans plusieurs incubateurs, de telle sorte que les supports de mesure (22) sont soumis à différents environnements d'incubation et mouvements du dispositif de déplacement (25).
